(19) Europäisches Patentamt
European Patent Office
Office européen des brevets

(11) EP 1 526 851 B1

(12) **EUROPEAN PATENT SPECIFICATION**

(45) Date of publication and mention of the grant of the patent:
**12.09.2007 Bulletin 2007/37**

(21) Application number: **03766118.8**

(22) Date of filing: **31.07.2003**

(51) Int Cl.:
***A61K 31/41*** *(2006.01)* ***A61P 35/00*** *(2006.01)*
***A61P 43/00*** *(2006.01)*

(86) International application number:
**PCT/DK2003/000518**

(87) International publication number:
**WO 2004/012733 (12.02.2004 Gazette 2004/07)**

(54) **COMPOUNDS USEFUL FOR THE TREATMENT OF DISEASES RESPONSIVE TO ANTIANGIOGENETIC THERAPY**

VERBINDUNGEN NÜTZLICH IN DER BEHANDLUNG VON ERKRANKUNGEN DIE AUF ANTI-ANGIOGENERTHERAPIE ANTWORTEN

COMPOSES UTILISES DANS LE TRAITEMENT DE MALADIES SENSIBLES A UNE THERAPIE ANTI-ANGIOGENETIQUE

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **01.08.2002 DK 200201165**
**28.11.2002 DK 200201839**
**11.03.2003 DK 200300371**

(43) Date of publication of application:
**04.05.2005 Bulletin 2005/18**

(73) Proprietor: **NEUROSEARCH A/S**
**2750 Ballerup (DK)**

(72) Inventors:
- **LICHTENBERG, Jens, DK-3230 Graested (DK)**
- **CHRISTOPHERSEN, Palle, NeuroSearch A/S DK-2750 Ballerup (DK)**
- **DAHL, Bjarne, H., NeuroSearch A/S DK-2750 Ballerup (DK)**

(56) References cited:
**WO-A-00/24707** **WO-A-00/76495**
**WO-A-96/25157** **WO-A-98/47879**
**WO-A-99/32436**

- **DATABASE BIOSIS [Online] BIOSCIENCES INFORMATION SERVICE, PHILADELPHIA, PA, US; December 2000 (2000-12) SHEN MENG-RU ET AL: "Differential expression of volume-regulated anion channels during cell cycle progression of human cervical cancer cells" Database accession no. PREV200100069159 XP002262442 & JOURNAL OF PHYSIOLOGY (CAMBRIDGE), vol. 529, no. 2, December 2000 (2000-12), pages 385-394, ISSN: 0022-3751**
- **VANGELIS G. MANOLOPOULOS ET AL: "Inhibition of angiogenesis by blockers of volume-regulated anion channels" GENERAL PHARMACOLOGY, vol. 34, 2000, pages 107-116, XP002262438**
- **A.J. NICHOLL ET AL: "The role of bicarbonate in regulatory volume decrease (RVD) in the epithelial-derived human breast cancer cell line ZR-75-1" PFLÜGERS ARCH - EUR J PHYSIOL, vol. 443, 2002, pages 875-881, XP002262439**
- **POUL BENNEKOU ET AL : "Treatment with NS3623, a novel Cl-conductance blocker, ameliorates erythrocyte dehydration in transgenic SAD mice: a possible new therapeutic approach for sickle cell disease." BLOOD, vol. 97, no. 5, 1 March 2001 (2001-03-01), pages 1451-1457, XP002262440**
- **ANTONIO R.T. ET AL: "Antiangiogenic and antiproliferative activity of suramin analogues" CANCER CHEMOTHER PHARMACOL, vol. 41, 1998, pages 117-124, XP002262441**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 526 851 B1

## Description

### TECHNICAL FIELD

This invention relates to the use of certain compounds for the treatment of diseases that are responsive to antiangiogenetic therapy, in particular for anti-metastatic treatment or for the treatment of age-related macular degeneration.

### BACKGROUND ART

Angiogenesis (or neovascularisation) is the formation of new blood vessels by sprouting from preexisting vessels. Angiogenesis is generally absent in healthy adult or mature tissue. However, it occurs in the healthy body for healing wounds and for restoring blood flow to tissues after injury or insult. In females, angiogenesis also occurs during the monthly reproductive cycle and during pregnancy. Under these processes, the formation of new blood vessels is strictly regulated.

In many serious disease states, the body loses control over angiogenesis. Excessive angiogenesis occurs in diseases such as cancer, diabetic blindness, age-related macular degeneration, rheumatoic arthritis, and psoriasis. In these conditions, new blood vessels feed diseased tissues, destroy normal tissues, and in the case of cancer, the new vessels allow tumor cells to escape into the circulation and lodge in other organs (tumor metastasis).

Experimental evidence has accumulated over the years to show that a variety of strategies that limit angiogenesis also slow or inhibit tumour growth, suggesting that blocking tumour-induced angiogenesis is a valid, novel approach to tumour therapy.

Age-related macular degeneration (AMD) is a common eye disease that gradually destroys central visual function. AMD occurs in stages from the early stage dry form to the later-stage and more severe wet form associated with the formation of new abnormal blood vessels in the back of the eye.

Thus there is a continued need for new antiangiogenetic therapies aiming at halting new blood vessel growth.

WO 98/47879 and WO 00/24707 (NeuroSearch A/S) describe a number of substituted phenyl derivatives active as chloride channel blockers.

WO 00/76495 (Smithkline Beecham Corp.) describes a number of substituted phenyl derivatives active as IL-8 receptor antagonists.

### SUMMARY OF THE INVENTION

It is an object of the invention to provide new therapies for treating diseases that are responsive to antiangiogenetic therapy. In particular, it is an object to provide therapies for arresting tumor growth and preventing the formation of metastases. A further object of the invention is the provision of therapies for treating age-related macular degeneration.

In its first aspect, the invention provides the use of a compound of general formula I

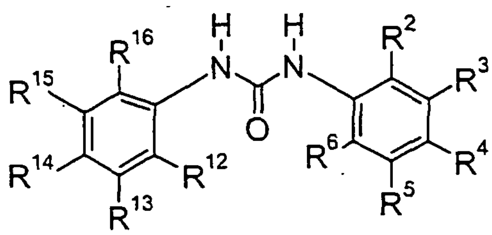

(I)

or a pharmaceutically acceptable salt thereof for the manufacture of a pharmaceutical composition for the treatment, prevention or alleviation of a disease or a disorder or a condition of a mammal, including a human, which disease, disorder or condition is responsive to inhibition of angiogenesis.

In a second aspect, the invention provides the use of a VRAC blocker or a pharmaceutically acceptable salt thereof for the manufacture of a pharmaceutical composition for the treatment, prevention or alleviation of age-related macular degeneration.

Other objects of the invention will be apparent to the person skilled in the art from the following detailed description and examples.

## DETAILED DISCLOSURE OF THE INVENTION

According to the invention it has now been found that certain compounds can be used for the treatment of diseases that are responsive to antiangiogenetic therapy, in particular for anti-metastatic treatment.

Thus, in its first aspect, the invention relates to the use of a compound of general formula I

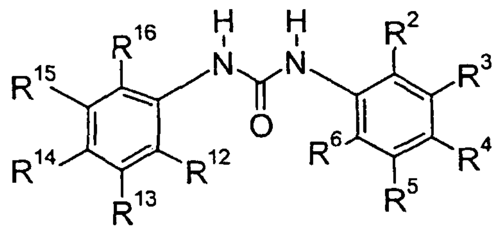

(I)

or a pharmaceutically acceptable salt thereof
wherein $R^2$ represents tetrazolyl; and

- $R^3$, $R^4$, $R^5$, $R^6$, $R^{12}$, $R^{13}$, $R^{14}$, $R^{15}$, and $R^{16}$ independently of each other represent hydrogen, halo, trifluoromethyl, nitro, alkyl, alkylcarbonyl, $-NR^aR^b$, $-NR^a-CO-R^b$, phenyl or heteroaryl;
  which phenyl is optionally substituted with halo, trifluoromethyl, nitro, $-CO-NHR^c$, $-CO-O-R^c$ or -CO-NR'R";
  wherein $R^c$ is hydrogen, alkyl, or phenyl;
  R' and R" independently of each other are hydrogen or alkyl; or
  R' and R" together with the nitrogen to which they are attached form a 5- to 7-membered heterocyclic ring, which ring may optionally comprise as a ring member, one oxygen atom, and/or one additional nitrogen atom, and/or one carbon-carbon double bond, and/or one carbon-nitrogen double bond;
  and which heterocyclic ring may optionally be substituted with alkyl;
  $R^a$ and $R^b$ independently of each other are hydrogen or alkyl; or

- $R^{15}$ and $R^{16}$, or $R^{14}$ and $R^{15}$ together with the phenyl ring to which they are attached form a naphthyl ring or an indanyl ring; and $R^3$, $R^4$, $R^5$, $R^6$, $R^{12}$ and $R^{13}$ and the remaining one of $R^{14}$, $R^{15}$ and $R^{16}$ are as defined above;

for the manufacture of a pharmaceutical composition for the treatment, prevention or alleviation of a disease or a disorder or a condition of a mammal, including a human, which disease, disorder or condition is responsive to inhibition of angiogenesis.

In a special embodiment, the invention relates to the use of a compound of general formula I

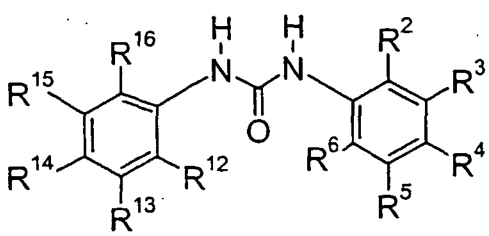

(I)

or a pharmaceutically acceptable salt thereof
wherein $R^2$ represents tetrazolyl;
$R^3$, $R^4$, $R^5$, $R^6$, $R^{12}$, $R^{13}$, $R^{14}$, $R^{15}$, and $R^{16}$ independently of each other represent hydrogen, halo, trifluoromethyl, nitro, or phenyl;
which phenyl is optionally substituted with halo, trifluoromethyl, nitro, or $-CO-NHR^c$; wherein $R^c$ is hydrogen, alkyl, or phenyl;
for the manufacture of a pharmaceutical composition for the treatment, prevention or alleviation of a disease or a disorder or a condition of a mammal, including a human, which disease, disorder or condition is responsive to inhibition of angiogenesis.

In another aspect, the invention relates to a method of treatment, prevention or alleviation of a disease or a

disorder or a condition of a living animal body, including a human, which disorder, disease or condition is responsive to inhibition of angiogenesis, comprising the step of administering to such a living animal body, including a human, in need thereof a therapeutically effective amount of a compound of general formula I

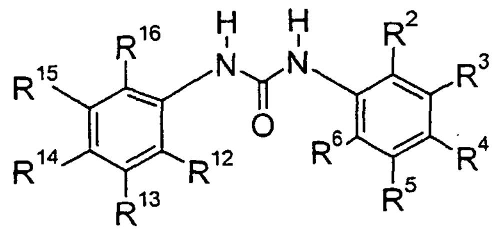

(I)

or a pharmaceutically acceptable salt thereof
wherein $R^2$ represents tetrazolyl; and

- $R^3$, $R^4$, $R^5$, $R^6$, $R^{12}$, $R^{13}$, $R^{14}$, $R^{15}$, and $R^{16}$ independently of each other represent hydrogen, halo, trifluoromethyl, nitro, alkyl alkylcarbonyl, $-NR^aR^b$, $-NR^a-CO-R^b$, phenyl or heteroaryl;
  which phenyl is optionally substituted with halo, trifluoromethyl, nitro, $-CO-NHR^c$, $-CO-O-R^c$ or -CO-NR'R";
  wherein $R^c$ is hydrogen, alkyl, or phenyl;
  R' and R" independently of each other are hydrogen or alkyl; or
  R' and R" together with the nitrogen to which they are attached form a 5- to 7-membered heterocyclic ring, which ring may optionally comprise as a ring member, one oxygen atom, and/or one additional nitrogen atom, and/or one carbon-carbon double bond, and/or one carbon-nitrogen double bond;
  and which heterocyclic ring may optionally be substituted with alkyl;
  $R^a$ and $R^b$ independently of each other are hydrogen or alkyl; or

- $R^{15}$ and $R^{16}$, or $R^{14}$ and $R^{15}$ together with the phenyl ring to which they are attached form a naphthyl ring or an indanyl ring; and $R^3$, $R^4$, $R^5$, $R^6$, $R^{12}$ and $R^{13}$ and the remaining one of $R^{14}$, $R^{15}$ and $R^{16}$ are as defined above.

In a special embodiment, the invention relates to a method of treatment, prevention or alleviation of a disease or a disorder or a condition of a living animal body, including a human, which disorder, disease or condition is responsive to inhibition of angiogenesis, comprising the step of administering to such a living animal body, including a human; in need thereof a therapeutically effective amount of a compound of general formula I

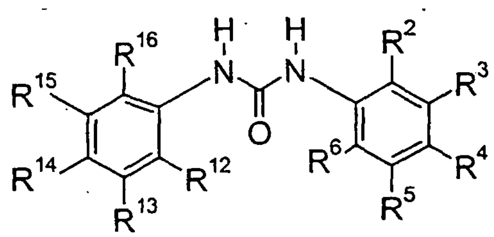

(I)

or a pharmaceutically acceptable salt thereof
wherein $R^2$ represents tetrazolyl;
$R^3$, $R^4$, $R^5$, $R^6$, $R^{12}$, $R^{13}$, $R^{14}$, $R^{15}$, and $R^{16}$ independently of each other represent hydrogen, halo, trifluoromethyl, nitro, or phenyl;
which phenyl is optionally substituted with halo, trifluoromethyl, nitro, or $-CO-NHR^c$;
wherein $R^c$ is hydrogen, alkyl, or phenyl.

The living animal body to be treated according to this invention is preferably a mammal, most preferably a human, in need for such treatment.

In one embodiment of the compound of general formula I, $R^2$ represents tetrazolyl; $R^3$, $R^4$, $R^5$, $R^6$, $R^{12}$, $R^{13}$, $R^{14}$, $R^{15}$, and $R^{16}$ independently of each other represent hydrogen, halo, trifluoromethyl, or nitro.

In a second embodiment of the compound of general formula I, $R^3$, $R^5$, and $R^6$ represent hydrogen; and $R^4$ represents halo, such as bromine.

In a third embodiment of the compound of general formula I, $R^3$, R4, $R^5$ and $R^6$ represent hydrogen.

In a further embodiment of the compound of general formula 1, $R^3$, $R^5$, and $R^6$ represent hydrogen; and $R^4$ represents $-NR^aR^b$, such as amino.

In a still further embodiment of the compound of general formula I, $R^3$, $R^5$, and $R^6$ represent hydrogen; and $R^4$ represents $-NR^a-CO-R^b$, such as acetylamino.

In a further embodiment of the compound of general formula I, $R^3$, $R^5$, and $R^6$ represent hydrogen; and $R^4$ represents phenyl substituted with trifluoromethyl, nitro or $-CO-NHR^c$; wherein $R^c$ is phenyl. In a special embodiment, $R^4$ represents phenyl substituted with trifluoromethyl, such as 4-trifluoromethylphenyl. In a further embodiment, $R^4$ represents phenyl substituted with nitro, such as 3-nitrophenyl. In a further embodiment, $R^4$ represents phenyl substituted with $-CO-NHR^c$, such as anilinocarbonylphenyl, in particular 4-anilinocarbonylphenyl.

In a further embodiment of the compound of general formula I, $R^3$, $R^5$, and $R^6$ represent hydrogen; and $R^4$ represents phenyl substituted with $-CO-O-R^c$ or -CO-NR'R". In a special embodiment, $R^4$ represents phenyl substituted with $-CO-O-R^c$, wherein $R^c$ is hydrogen. In a special embodiment, $R^4$ represents phenyl substituted with -CO-NR'R", such as 4-dimethylcarbamoylphenyl or 4-(4-methyl-1-piperazine-carbonyl)-phenyl.

In a still further embodiment, $R^{15}$ represents trifluoromethyl. In a special embodiment, $R^{15}$ represents trifluoromethyl and $R^{12}$, $R^{13}$, $R^{14}$ and $R^{16}$ represent hydrogen. In a further embodiment $R^{13}$ represents trifluoromethyl. In a special embodiment, $R^{13}$ and $R^{15}$ represent trifluoromethyl and $R^{12}$, $R^{14}$ and $R^{16}$ represent hydrogen.

In a further embodiment, $R^{16}$ represents trifluoromethyl. In a special embodiment, $R^{16}$ represents trifluoromethyl and $R^{12}$, $R^{13}$, $R^{14}$ and $R^{15}$ represent hydrogen.

In a still further embodiment, $R^{15}$ represents halo, such as chloro or bromo. In a special embodiment, $R^{15}$ represents halo, such as chloro or bromo, and $R^{12}$, $R^{13}$, $R^{14}$ and $R^{16}$ represent hydrogen. In a further embodiment, $R^{13}$ and $R^{15}$ represent halo, such as chloro, and $R^{12}$, $R^{14}$ and $R^{16}$ represent hydrogen. In a still further embodiment, $R^{14}$ and $R^{15}$ represent halo, such as chloro, and $R^{12}$, $R^{13}$ and $R^{16}$ represent hydrogen.

In a further embodiment, $R^{16}$ represents halo, such as fluoro. In a special embodiment, $R^{16}$ represents halo, such as fluoro, and $R^{12}$, $R^{13}$, $R^{14}$ and $R^{15}$ represent hydrogen.

In a still further embodiment, $R^{16}$ represents alkyl, such as methyl or ethyl. In a special embodiment, $R^{16}$ represents alkyl, such as methyl or ethyl, and $R^{12}$, $R^{13}$, $R^{14}$ and $R^{15}$ represent hydrogen.

In a further embodiment, $R^{14}$ represents nitro. In a special embodiment, $R^{14}$ represents nitro, and $R^{12}$, $R^{13}$, $R^{15}$ and $R^{16}$ represent hydrogen.

In a still further embodiment, $R^{14}$ represents alkylcarbonyl, such as acetyl. In a special embodiment, $R^{14}$ represents alkylcarbonyl, such as acetyl, and $R^{12}$, $R^{13}$, $R^{15}$ and $R^{16}$ represent hydrogen.

In a further embodiment, $R^{15}$ represents phenyl. In a further embodiment, $R^{14}$ represents phenyl. In a special embodiment, one of $R^{14}$ or $R^{15}$ represents phenyl, and the remaining of $R^{12}$, $R^{13}$,$R^{14}$, $R^{15}$ and $R^{16}$ represent hydrogen.

In a still further embodiment, $R^{15}$ represents pyridyl, such as pyridin-3-yl. In a special embodiment, $R^{15}$ represents pyridyl, such as pyridin-3-yl, and $R^{12}$, $R^{13}$, $R^{14}$ and $R^{16}$ represent hydrogen.

In a further embodiment, $R^{15}$ and $R^{16}$ together with the phenyl ring to which they are attached form a naphthyl ring.

In a still further embodiment, $R^{14}$ and $R^{15}$ together with the phenyl ring to which they are attached form an indanyl ring.

In a further embodiment, the compound of general formula I is

*N*-4-Nitrophenyl-*N'*-[4-bromo-2-(1-*H*-tetrazol-5-yl)phenyl] urea;
*N*-3,5-Di(trifluoromethyl)phenyl-*N'*-[4-bromo-2-(1-*H*-tetrazol-5-yl)phenyl] urea;
*N*-3-Trifluoromethylphenyl-*N*'-[4-(3-nitrophenyl)-2-(1-*H*-tetrazol-5-yl)phenyl] urea;
*N*-3-Trifluoromethylphenyl-*N*'-[4-(4-anilinocarbonylphenyl)-2-(1-*H*-tetrazol-5-yl)phenyl] urea;
*N*-3-Trifluoromethylphenyl-*N*'-[4-(4-trifluoromethylphenyl)-2-(1-*H*-tetrazol-5-yl)phenyl] urea;
*N*-(3-Trifluoromethyl-phenyl)-*N*'-[2-(1-*H*-tetrazol-5-yl)-phenyl] urea;
*N*-(3-Trifluoromethyl-phenyl)-*N*'-[4-bromo-2-(1-*H*-terazol-5-yl)-phenyl] urea;
*N*-(3-Trilfuoromethyl-phenyl)-*N*'-[4-phenyl-2-(1-*H*-tetrazol-5-yl)-phenyl] urea;
*N*-(3-Chloro-phenyl)-*N*'-[2-(1-*H*-tetrazol-5-yl)-phenyl] urea;
*N*-(3-Trifluoromethyl-phenyl)-*N*'-[4-amino-2-(1-*H*-tetrazol-5-yl)-phenyl] urea;
*N*-(3-Trifluoromethyl-phenyl)-*N*'-[4-acetylamino-2-(1-*H*-tetrazol-5-yl)-phenyl] urea;
*N*-(3-Trilfuoromethyl-phenyl)-*N*'-[4-carbamoyl-2-(1-*H*-tetrazol-5-yl)-phenyl] urea;
*N*-(3-Trifluoromethyl-phenyl)-*N*'-[4-(*N*",*N*"-dimethylcarbamoyl)-2-(1-*H*-tetrazol-5-yl)-phenyl] urea;
3'-(1-*H*-tetrazol-5-yl)-4'-[3-(3-trifluoromethyl-phenyl)-ureido]-biphenyl-4-carboxylic acid;
*N*-(Indan-5-yl)-*N*'-[2-(1-*H*-tetrazol-5-yl)-phenyl] urea;
*N*-(Biphenyl-4-yl)-*N*'-[2-(1-*H*-tetrazol-5-yl)-phenyl] urea;
*N*-(Biphenyl-3-yl)-*N*'-[2-(1-*H*-tetrazol-5-yl)-phenyl] urea;

*N*-(3-Acetyl-phenyl)-*N*'-[2-(1-*H*-tetrazol-5-yl)-phenyl] urea;
*N*-(Biphenyl-3-yl)-*N'*-[2-(1-*H*-tetrazol-5-yl)-phenyl] urea;
*N*-[3-(Pyridin-3-yl)-phenyl]-*N'*-[2-(1-*H*-tetrazol-5-yl)-phenyl] urea;
*N*-(3-Bromo-phenyl)-*N'*-[4'-(4-methyl-piperazine-1-carbonyl)-3-(1-*H*-tetrazol-5-yl)-biphenyl-4-yl] urea;
*N*-(3,5-Dichloro-phenyl)-*N'*-[4-bromo-2-(1-*H*-tetrazol-5-yl)-phenyl] urea;
*N*-(3,4-Dichloro-phenyl)-*N'*-[4-bromo-2-(1-*H*-tetrazol-5-yl)-phenyl] urea;
*N*-(Naphthalen-1-yl)-*N'*-[4-bromo-2-(1-*H*-tetrazol-5-yl)-phenyl] urea;
*N*-(2-Trifluoromethyl-phenyl)-*N'*-[4-bromo-2-(1-*H*-tetrazol-5-yl)-phenyl] urea;
*N*-(2-Fluoro-phenyl)-*N'*-[4-bromo-2-(1-*H*-tetrazol-5-yl)-phenyl] urea;
*N*-(2-Ethyl-phenyl)-*N'*-[4-bromo-2-(1-*H*-tetrazol-5-yl)-phenyl] urea;

or a pharmaceutically acceptable salt thereof.

In a still further embodiment, the disease, disorder or condition that is responsive to inhibition of angiogenesis is selected from the group consisting of cancer, prostate cancer, lung cancer, breast cancer, bladder cancer, renal cancer, colon cancer, gastric cancer, pancreatic cancer, ovarian cancer, melanoma, hepatoma, sarcoma, lymphoma, exudative macular degeneration, age-related macular degeneration, retinopathy, diabetic, proliferative diabetic retinopathy, diabetic macular edema (DME), ischemic retinopathy, retinopathy of prematurity, neovascular glaucoma, corneal neovascularization, rheumatoid arthritis, and psoriasis.

In a special embodiment of the invention, the compound is

*N*-4-Nitrophenyl-*N*'-[4-bromo-2-(1-*H*-tetrazol-5-yl)phenyl] urea;
N-3,5-Di(trifluoromethyl)phenyl-*N*'-[4-bromo-2-(1-*H*-tetrazol-5-yl)phenyl] urea;
*N*-3-Trifluoromethylphenyl-*N'*-[4-(3-nitrophenyl)-2-(1-*H*-tetrazol-5-yl)phenyl] urea;
*N*-3-Trifluoromethylphenyl-*N'*-[4-(4-anilinocarbonylphenyl)-2-(1-*H*-tetrazol-5-yl)phenyl]urea;
*N*-3-Trifluoromethylphenyl-*N'*-[4-(4-trifluoromethylphenyl)-2-(1-*H*-tetrazol-5-yl)phenyl] urea;
or a pharmaceutically acceptable salt thereof, and

the treatment is an anti-metastatic treatment.

In a further aspect, the invention relates to the use of a VRAC blocker or a pharmaceutically acceptable salt thereof for the manufacture of a pharmaceutical composition for the treatment, prevention or alleviation of age-related macular degeneration of a mammal, including a human.

In a still further aspect the invention relates to a method of treatment, prevention or alleviation of age-related macular degeneration of a living animal body, including a human comprising the step of administering to such a living animal body, including a human, in need thereof a therapeutically effective amount of a VRAC blocker or a pharmaceutically acceptable salt thereof.

In one embodiment, the VRAC blocker is a compound of general formula I

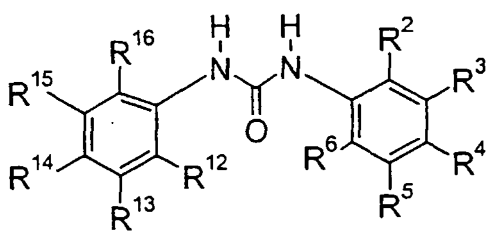

(I)

or a pharmaceutically acceptable salt thereof
wherein $R^2$ represents tetrazolyl; and

- $R^3$, $R^4$, $R^5$, $R^6$, $R^{12}$, $R^{13}$, $R^{14}$, $R^{15}$,and $R^{16}$ independently of each other represent hydrogen, halo, trifluoromethyl, nitro, alkyl, alkylcarbonyl, $-NR^aR^b$, $-NR^a-CO-R^b$, phenyl or heteroaryl;
  which phenyl is optionally substituted with halo, trifluoromethyl, nitro, $-CO-NHR^c$, $-CO-O-R^c$ or -CO-NR'R";
  wherein $R^c$ is hydrogen, alkyl, or phenyl;
  R' and R" independently of each other are hydrogen or alkyl; or
  R' and R" together with the nitrogen to which they are attached form a 5- to 7-membered heterocyclic ring, which ring may optionally comprise as a ring member, one oxygen atom, and/or one additional nitrogen atom, and/or one carbon-carbon double bond, and/or one carbon-nitrogen double bond;

and which heterocyclic ring may optionally be substituted with alkyl;
$R^a$ and $R^b$ independently of each other are hydrogen or alkyl; or

- $R^{15}$ and $R^{16}$, or $R^{14}$ and $R^{15}$ together with the phenyl ring to which they are attached form a naphthyl ring or an indanyl ring; and $R^3$, $R^4$, $R^5$, $R^6$, $R^{12}$ and $R^{13}$ and the remaining one of $R^{14}$, $R^{15}$ and $R^{16}$ are as defined above;

for the manufacture of a pharmaceutical composition for the treatment, prevention or alleviation of a disease or a disorder or a condition of a mammal, including a human, which disease, disorder or condition is responsive to inhibition of angiogenesis.

In a further embodiment, the VRAC blocker is a compound of general formula I

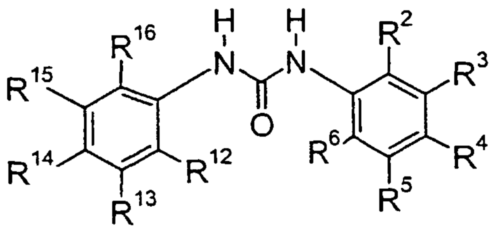

(I)

or a pharmaceutically acceptable salt thereof
wherein $R^2$ represents tetrazolyl;
$R^3$, $R^4$, $R^5$, $R^6$, $R^{12}$, $R^{13}$, $R^{14}$, $R^{15}$, and $R^{16}$ independently of each other represent hydrogen, halo, trifluoromethyl, nitro, or phenyl;
which phenyl is optionally substituted with halo, trifluoromethyl, nitro, or -CO-NHR$^a$;
wherein $R^a$ is hydrogen, alkyl, or phenyl.

In a special embodiment, the VRAC blocker is

*N*-4-Nitrophenyl-*N*'-[4-bromo-2-(1-*H*-tetrazol-5-yl)phenyl] urea;
*N*-3,5-Di(trifluoromethyl)phenyl-*N*'-[4-bromo-2-(1-*H*-tetrazol-5-yl)phenyl] urea;
*N*-3-Trifluoromethylphenyl-*N*'-[4-(3-nitrophenyl)-2-(1-*H*-tetrazol-5-yl)phenyl] urea;
*N*-3-Trifluoromethylphenyl-*N'*-[4-(4-anilinocarbonylphenyl)-2-(1-*H*-tetrazol-5-yl)phenyl] urea;
*N*-3-Trifluoromethylphenyl-*N*'-[4-(4-trifluoromethylphenyl)-2-(1-*H*-tetrazol-5-yl)phenyl] urea;
*N*-(3-Trifluoromethyl-phenyl)-*N*'-[2-(1-*H*-tetrazol-5-yl)-phenyl] urea;
*N*-(3-Trifluoromethyl-phenyl)-*N*'-[4-bromo-2-(1-*H*-terazol-5-yl)-phenyl] urea;
*N*-(3-Trilfuoromethyl-phenyl)-*N*'-[4-phenyl-2-(1-*H*-tetrazol-5-yl)-phenyl] urea;
*N*-(3-Chloro-phenyl)-*N*'-[2-(1-*H*-tetrazol-5-yl)-phenyl] urea;
*N*-(3-Trifluoromethyl-phenyl)-*N*'-[4-amino-2-(1-*H*-tetrazol-5-yl)-phenyl] urea;
*N*-(3-Trifluoromethyl-phenyl)-*N'*-[4-acetylamino-2-(1-*H*-tetrazol-5-yl)-phenyl] urea;
*N*-(3-Trilfuoromethyl-phenyl)-*N'*-[4-carbamoyl-2-(1-*H*-tetrazol-5-yl)-phenyl] urea;
*N*-(3-Trifluoromethyl-phenyl)-*N'*-[4-(*N''*,*N''*-dimethylcarbamoyl)-2-(1-*H*-tetrazol-5-yl)-phenyl] urea;
3'-(1-*H*-tetrazol-5-yl)-4'-[3-(3-trifluoromethyl-phenyl)-ureido]-biphenyl-4-carboxylic acid;
*N*-(Indan-5-yl)-*N'*-[2-(1-*H*-tetrazol-5-yl)-phenyl] urea;
N-(Biphenyl-4-yl)-*N'*-[2-(1-*H*-tetrazol-5-yl)-phenyl] urea;
*N*-(Biphenyl-3-yl)-*N'*-[2-(1-*H*-tetrazol-5-yl)-phenyl] urea;
*N*-(3-Acetyl-phenyl)-*N'*-[2-(1-*H*-tetrazol-5-yl)-phenyl] urea;
*N*-(Biphenyl-3-yl)-*N'*-[2-(1-*H*-tetrazol-5-yl)-phenyl] urea;
*N*-[3-(Pyridin-3-yl)-phenyl]-*N*'-[2-(1-*H*-tetrazol-5-yl)-phenyl] urea;
*N*-(3-Bromo-phenyl)-*N*'-[4'-(4-methyl-piperazine-1-carbonyl)-3-(1-*H*-tetrazol-5-yl)-biphenyl-4-yl] urea;
*N*-(3,5-Dichloro-phenyl)-*N*'-[4-bromo-2-(1-*H*-tetrazol-5-y1)-phenyl] urea;
*N*-(3,4-Dichloro-phenyl)-*N*'-[4-bromo-2-(1-*H*-tetrazol-5-yl)-phenyl] urea;
*N*-(Naphthalen-1-yl)-*N*'-[4-bromo-2-(1-*H*-tetrazol-5-yl)-phenyl] urea;
*N*-(2-Trifluoromethyl-phenyl)-*N*'-[4-bromo-2-(1-*N*-tetrazol-5-yl)-phenyl] urea;
*N*-(2-Fluoro-phenyl)-*N'*-[4-bromo-2-(1-*H*-tetrazol-5-yl)-phenyl] urea;
*N*-(2-Ethyl-phenyl)-*N'*-[4.-bromo-2-(1-*H*-tetrazol-5-yl)-phenyl] urea;

or a pharmaceutically acceptable salt thereof.

Definition of Substituents

In the context of this invention halo represents fluoro, chloro, bromo or iodo.

In the context of this invention an alkyl group designates a univalent saturated, straight or branched hydrocarbon chain. The hydrocarbon chain preferably contain of from one to six carbon atoms ($C_{1-6}$-alkyl), including pentyl, isopentyl, neopentyl, tertiary pentyl, hexyl and isohexyl. In one embodiment alkyl represents a $C_{1-4}$-alkyl group, including butyl, isobutyl, secondary butyl, and tertiary butyl. In another embodiment of this invention alkyl represents a $C_{1-3}$-alkyl group, which may in particular be methyl, ethyl, propyl or isopropyl.

In the context of this invention a heteroaryl group designates an aromatic mono-, bi-or poly-heterocyclic group, which holds one or more heteroatoms in its ring structure. Preferred heteroatoms include nitrogen (N), oxygen (O), and sulphur (S). Preferred monocyclic heteroaryl groups of the invention include aromatic 5- and 6 membered heterocyclic monocyclic groups, including furanyl, in particular 2- or 3-furanyl; thienyl, in particular 2 or 3-thienyl; pyrrolyl (azolyl), in particular 1,2 or 3-pyrrolyl; oxazolyl, in particular oxazol-2,4 or 5-yl; thiazolyl, in particular thiazol-2,4 or 5-yl; imidazolyl, in particular 1,2 or 4-imidazolyl; pyrazolyl, in particular 1,3 or 4-pyrazolyl; isoxazolyl, in particular isoxazol-3,4 or 5-yl; isothiazolyl, in particular isothiazol-3,4 or 5-yl; oxadiazolyl, in particular 1,2,3-, 1,2,4-, 1,2,5- or 1,3,4-oxadiazol-3,4 or 5-yl; triazolyl, in particular 1,2,3-, 1,2,4-, 2,1,3- or 4,1,2-triazolyl; thiadiazolyl, in particular thiadiazol-3,4 or 5-yl; pyridinyl, in particular 2,3 or 4-pyridinyl; pyridazinyl, in particular 3 or 4-pyridazinyl; pyrimidinyl, in particular 2,4 or 5-pyrimidinyl; pyrazinyl, in particular 2 or 3-pyrazinyl; and triazinyl, in particular 1,2, 3-, 1,2,4-or 1,3,5-triazinyl.

5- to 7-membered heterocyclic rings comprising one nitrogen atom include for example, but not limited to, pyrolidine, piperidine, homopiperidine, pyrroline, tetrahydropyridine, pyrazolidine, imidazolidine, piperazine, homopiperazine, and morpholine.

VRAC

Volume regulated anion channels (VRAC) are present in most mammalian cells. An important function of VRAC is cell volume regulation: Upon swelling of the cell in hypotonic solution these channels are activated and chloride ions flow out of the cell in parallel with potassium ions (via potassium channels) and water, thereby restoring the original cell volume.

Although VRAC has not been cloned and therefore cannot yet be defined by their gene sequences, there is a number of defining characteristics for these channels as observed with the whole cell patch clamp technique: VRAC is activated by cell swelling (hypotonic extracellular - or hypertonic intracellular media) and an important trigger for activation is lowered intracellular ionic strength rather than volume per se. Activation of VRAC is obligatory dependent on the presence of intracellular ATP and complex intracellular signalling cascades involving protein kinases support the activation process. VRAC. is an voltage independent, outward rectifying anion selective channel exhibiting type I Eisenman halide selectivity sequence (implying for example that iodide is more permeable than chloride) and having a wide pore that also allows the permeation of a large number of negatively charged or neutral organic molecules. VRAC is always blocked voltage-dependently by DIDS, and NPPB and tamoxifen are voltage independent blockers of this channel.

VRAC blockers

A VRAC blocker is a compound that inhibits the transmembrane transport of chloride or any other anion or neutral molecule in response to cell swelling or decrease in intracellular ionic strength.

The potential of a given substance to act as a VRAC blocker may be determined using standard laboratory test methods, such as

a) whole cell or single channel patch clamp technology,
b) microelectrode electrophysiology (penetrating, sharp electrodes),
c) flux assays (e.g. radioactive as well as non-radioactive isotopes),
d) fluorescent dyes (e.g. membrane potential - or chloride concentration indicators),
e) cell volume measurements (e.g. light scattering, coulter counter measurements, or optical methods such as image analysis)

The VRAC blocker may in particular be a diphenyl urea derivative such as those disclosed in described in WO 98/47879 or WO 00/24707 (NeuroSearch A/S).

In one embodiment, the VRAC blockers show $IC_{50}$-values of less than 10 μM, preferably less than 1000 nM, more preferably less than 100 nM, even more preferably less than 50 nM, and most preferred less than 10 nM for *in*

*vitro* inhibition according to the standard test methods.

In a second embodiment the VRAC blockers show $ED_{50}$ values of less than 50 mg/kg, preferably less than 10 mg/kg, more preferably less than 5 mg/kg in the standard *in vivo* angiogenesis models.

Methods of Preparation

The compounds for use according to the invention may be prepared by conventional methods for chemical synthesis, e.g. those described in WO 98/47879 or WO 00/24707 (NeuroSearch A/S).

Pharmaceutically Acceptable Salts

The active compound for use according to the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulphonate derived from benzensulphonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulphonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound for use according to the invention and its pharmaceutically acceptable acid addition salt.

Examples of pharmaceutically acceptable cationic salts of a chemical compound of the invention include, without limitation, the sodium, the potassium, the calcium, the magnesium, the zinc, the aluminium, the lithium, the choline, the lysine, and the ammonium salt, and the like, of a chemical compound of the invention containing an anionic group. Such cationic salts may be formed by procedures well known and described in the art.

In the context of this invention the "onium salts" of N-containing compounds are also contemplated as pharmaceutically acceptable salts (aza-onium salts). Preferred azaonium salts include the alkyl-onium salts, in particular the methyl- and the ethyl-onium salts; the cycloalkyl-onium salts, in particular the cyclopropyl-onium salts; and the cycloalkylalkyl-onium salts, in particular the cyclopropyl-methyl-onium salts.

Pharmaceutical Compositions

While the active compound for use in therapy according to the invention may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the chemical compound for use according to the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof. In a further embodiment, the invention provides pharmaceutical compositions comprising more than one compound or prodrug for use according to the invention, such as two different compounds or prodrugs for use according to the invention.

Pharmaceutical compositions of the invention may be those suitable for oral, rectal, bronchial, nasal, pulmonal, topical (including buccal and sub-lingual), transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of

solid hydrophobic polymers containing the compound of the invention, which matrices may be in form of shaped articles, e.g. films or microcapsules.

The chemical compound of the invention, together with a conventional adjuvant, carrier, or diluent, may thus be placed into the form of pharmaceutical compositions and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The chemical compound of the present invention can be administered in a wide variety of oral and parenteral dosage forms. It will be obvious to those skilled in the art that the following dosage forms may comprise, as the active component, either a chemical compound of the invention or a pharmaceutically acceptable salt of a chemical compound of the invention.

For preparing pharmaceutical compositions from a chemical compound of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material..

In powders, the carrier is a finely divided solid, which is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify.

Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations, intended for conversion shortly before use to liquid form preparations for oral administration. Such liquid forms include solutions, suspensions, and emulsions. In addition to the active component such preparations may comprise colorants, flavours; stabilisers, buffers, artificial and natural sweeteners, dispersants, thickeners, solubilizing agents, and the like.

For topical administration to the epidermis the chemical compound of the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing

agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co, Easton, PA).

A therapeutically effective dose refers to that amount of active ingredient, which ameliorates the symptoms or condition. Therapeutic efficacy and toxicity, e.g. $ED_{50}$ and $LD_{50}$, may be determined by standard pharmacological procedures in cell cultures or experimental animals. The dose ratio between therapeutic and toxic effects is the therapeutic index and may be expressed by the ratio $LD_{50}/ED_{50}$. Pharmaceutical compositions exhibiting large therapeutic indexes are preferred.

The dose administered must of course be carefully adjusted to the age, weight and condition of the individual being treated, as well as the route of administration, dosage form and regimen, and the result desired, and the exact dosage should of course be determined by the practitioner.

The actual dosage depend on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 1000 mg of active ingredient per individual dose, preferably of from about 1 to about 100 mg, are suitable for therapeutic treatments.

The active ingredient may be administered in one or several doses per day. Preferred ranges are from 10-200 mg/day p.o. administered in one or two doses, such as from 25-50 mg p.o. twice a day.

## Ophthalmic formulations

The pharmaceutical composition may be prepared in unit dosage forms suitable for topical ocular use. The therapeutically efficient amount typically is between 0.0001 and 5% (w/v), preferably between 0.001 and 1.0% (w/v) in liquid formulations.

For ophthalmic application, preferably solutions are prepared using a physiological saline solution as a major vehicle. The pH of such ophthalmic solutions should preferably be maintained between 4.5 and 8.0, more preferably between 6.5 and 7.2, with an appropriate buffer system. The formulations may also contain conventional, pharmaceutically acceptable preservatives, stabilizers and surfactants.

The preservative may be selected from hydrophobic or non-ionic preservatives, anionic preservatives, and cationic preservatives. Preferred preservatives that may be used in the pharmaceutical compositions of the present invention include, but are not limited to, benzalkonium chloride, chlorobutanol, thimerosal, phenylmercuric acetate and

phenylmercuric nitrate.

A preferred surfactant is, for example, Polysorbate 80. Likewise, various preferred vehicles may be used in the ophthalmic preparations of the present invention. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors, such as non-ionic tonicity adjustors, may be added as needed or convenient. They include, but are not limited to, salts, particularly sodium chloride, potassium chloride, mannitol and glycerol, polyethylene glycols (PEG), polypropylene glucols (PPG) or any other suitable ophthalmically acceptable tonicity adjustor.

Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed.

An ophthalmically acceptable antioxidant for use in the present invention includes, but is not limited to, sodium metabisulfite, sodium thiosulfate, acetylcysteine, butylated hydroxyanisole and butylated hydroxytoluene.

Other excipient components which may be included in the ophthalmic preparations are chelating agents. The preferred chelating agent is edentate disodium, although other chelating agents may also be used in place or in conjunction with it.

The ingredients are usually used in the following amounts:

| Ingredient | Amount (% w/v) |
|---|---|
| Active compound | 0.001-5 |
| Preservative | 0-0.10 |
| Vehicle | 0-40 |
| Tonicity adjustor | 1-10 |
| Buffer | 0.01-10 |
| pH adjustor | q.s. pH 4.5-8.0 |
| Antioxidant | as needed |
| Surfactant | as needed |
| Purified water | as needed to make 100% |

The actual dose of the active compounds of the present invention depends on the specific compound, and on the condition to be treated; the selection of the appropriate dose is well within the knowledge of the skilled artisan.

The ophthalmic formulations of the present invention are conveniently packaged in forms suitable for metered application, such as in containers equipped with a dropper, to facilitate the application to the eye. Containers suitable for dropwise application are usually made of suitable inert, non-toxic plastic material, and generally contain between about 0.5 and about 15 ml solution.

In the case of treating ophthalmic angiogenesis related diseases, disorders or conditions - such as AMD, the pharmaceutical composition of the invention may also be administered in the form of systemic administration (such as orally), as an eye ointment, or as an injection in the eye (periocular or intraocular injection).

**Methods of therapy**

The invention also provides a method for the treatment, prevention or alleviation of a disease or a disorder or a condition of a living animal body, including a human, which disease, disorder or condition is responsive to inhibition of angiogenesis, and which method comprises administering to such a living animal body, including a human, in need thereof an effective amount of a compound of general formula I as defined above.

The diseases, disorders or conditions that are responsive to inhibition of angiogenesis include but are not limited to:

- diseases, disorders or conditions that involve the proliferation of tumor cells, such as cancer, prostate cancer, lung cancer, breast cancer, bladder cancer, renal cancer, colon cancer, gastric cancer, pancreatic cancer, ovarian cancer, melanoma, hepatoma, sarcoma and lymphoma;
- ophthalmic angiogenesis related diseases, disorders or conditions, such as exudative macular degeneration, age-related macular degeneration (AMD), retinopathy, diabetic retinopathy, proliferative diabetic retinopathy, diabetic macular edema (DME), ischemic retinopathy (e.g. retinal vain or artery occlusion), retinopathy of prematurity, neovascular glaucoma, and corneal neovascularization; and

- rheumatoid arthritis, and psoriasis.

In a special embodiment, the disease, disorder or condition to be treated is a preneoplastic disease state. In a further embodiment, the treatment is an anti-metastatic treatment. In a still further embodiment, the disease, disorder or condition to be prevented is metastatic cancer. In a further embodiment, the disease, disorder or condition to be prevented or alleviated is DME.

Also the invention provides a method of treatment, prevention or alleviation of age-related macular degeneration of a living animal body, including a human, which method comprises administering to such a living animal body, including a human, in need thereof a therapeutically effective amount of a VRAC blocker or a pharmaceutically acceptable salt thereof.

In the context of this invention, "age-related macular degeneration" (AMD) includes dry AMD (non-exudative AMD) and wet AMD (exudative AMD).

In a special embodiment, the invention relates to treatment, prevention or alleviation of wet AMD.

**Combined therapy**

The pharmaceutical composition for use according to the invention may include or may be used or administered in combination with one or more additional drugs useful for the treatment, prevention or alleviation of a disease responsive to inhibition of angiogenesis, such as compounds useful for anti-metastatic treatment. Such additional drugs include cytotoxic compounds, antimitotic compounds, and antimetabolites.

Examples of cytotoxic compounds (including cytotoxic alkylating agents) include carmustine (BCNU), fotemustin, temozolomide (temodal), ifosfamide, and cyclofosfamide.

Examples of antimitotic compounds include paclitaxel (taxol) and docetaxel.

An example of antimetabolites includes methotrexat.

Furthermore, the pharmaceutical composition for use according to the invention may be used or administered in combination with other treatments or therapies. Examples of other treatments or therapies include radiotherapy and surgery.

**Test methods**

The efficacy of use of the compound according to the invention may be evaluated by standard *in vitro* and *in vivo* studies as e.g. those described below.

**In vitro methods**

*Cell-specificity assay: Incorporation of [$^3$H]thymidine*

Confluent cultures of HUVEC, fibroblasts, Mel 57 and T47D cells were detached by trypsin/EDTA solution, and allowed to adhere and spread at an appropriate cell density on gelatine-coated dishes in M199-HEPES medium or DMEM-HEPES medium both supplemented with 10% heat-inactivated new born calve serum (NBCS) and penicillin/streptomycin. After 18 h the HUVEC and fibroblasts were stimulated with 2.5 ng/ml FGF-2 in M199-HEPES, penicillin/streptomycin, 10% NBCS and 0.1 % DMSO in duplicate wells, with or without the test compounds. The tumor cells were cultured in DMEM-HEPES supplemented with 10% NBCS, penicillin/streptomycin and 0.1% DMSO in duplicate wells, with or without the test compounds. After an incubation period of 48 h, a tracer amount (0.5 $\mu$Ci/well) of [$^3$H]thymidine was added and the cells were incubated for another 6 h period. Subsequently, the cells were washed with PBS, [3H]-labelled DNA were fixed with methanol, and precipitated in 5% trichloroacetic-acid, and finally dissolved in 0.5 ml 0.3 M NaOH and counted in a liquid scintillation counter.

*Cell morphology and proliferation assay*

One week before the assay, a vial with HUVEC (passage 1) was thawed and cultured (after a split ratio of 1: 3) to confluence (passage 2). The confluent culture of HUVEC was detached by trypsin/EDTA solution, and allowed to adhere and spread at cell density of 10, 50 and 100% confluency on gelatine-coated dishes in M199-HEPES medium supplemented with 10% heat-inactivated NBCS, 10% human serum and penicillin/streptomycin. After 18 h the HUVEC were preincubated with the test compounds for 4 hours. Then the HUVEC were washed and restimulated in the absence or presence of the test compounds and the reference compounds with 2.5 ng/ml FGF-2 in M199-HEPES, penicillin/streptomycin, 10% NBCS, 10% human serum and 0.1% DMSO in triplicate wells for 5 (10% confluency) or 3 days (50 and 100% confluency), with or without the test compounds. The cell number was determined by image analysis (P.

Koolwijk, 2001)

*Observations analyses and measurements*

All outcome measures were measured in singular, i.e. one measurement per culture well. The proliferation of HUVEC, fibroblast, Mel 57, and T47D tumor cells was expressed as mean ± range [3H]-thymidine incorporation (dpm) of duplicate wells.

The percentage of inhibition of FGF-2-induced HUVEC and fibroblast proliferation by the compounds was calculated as follows:

$$\%\text{ inhibition} = \left(1 - \frac{((\text{dpm}, \text{HUVEC}_{\text{FGF-2}}) - (\text{dpm}, \text{HUVEC}_{\text{FGF-2 + compound}}))}{((\text{dpm}, \text{HUVEC}_{\text{FGF-2}}) - (\text{dpm}, \text{HUVEC}_{\text{control}}))}\right) \times 100\%$$

| | |
|---|---|
| $\text{HUVEC}_{\text{control}}$ | = non stimulated HUVEC |
| $\text{HUVEC}_{\text{FGF-2}}$ | = FGF-2 stimulated HUVEC |
| $\text{HUVEC}_{\text{FGF-2 + compound}}$ | = FGF-2 stimulated HUVEC + test compound |

The percentage of inhibition of the Mel 57 and T47D tumor cell proliferation by the compounds will be calculated as follows:

$$\%\text{ inhibition} = \left(1 - \frac{((\text{dpm, tumor cell}) - (\text{dpm, tumor cell}_{\text{+ compound}}))}{(\text{dpm, tumor cell})}\right) \times 100\%$$

| | |
|---|---|
| tumor cell | = NBCS stimulated tumor cell |
| $\text{tumor cell}_{\text{+ compound}}$= | NBCS stimulated tumor cell + test compound |

**In vivo methods**

*Mouse anti-angiogenesis assay*

NMRI female mice (SPF Bom:NMRI) weighing 25-27 g were obtained from M&B, Ejby, Lille Skensved, Denmark. They were housed in a facility where light was controlled on a 12 hour light-dark cycle. The room temperature and relative humidity recorded by a thermo-hygrograph showed values between 20.5-24.1 °C and 40-67%, respectively. The animals were fed a pelleted rodent diet (Altromin 1324, Brogården, Denmark) *ad libitum* and had free access to tap water. All animals were observed daily for clinical signs.

Slow-release pellets containing 400 ng of human basic fibroblast growth factor (Innovative Research of America, Florida, USA) were circular and with a diameter of 1.5 mm. The angiogenic peptide was guarantied by the supplier to be released over a period of 10 days.

The mice were anaesthetized using inhalation anaesthesia (halothane/$N_2O$ and oxygen). The skin of the back was shaved using an electric shaver and the skin was disinfected using 70% ethanol. A 5 mm incision was made transversely in close proximity to the shoulder blades and a 2 cm pocket reaching caudally to the pelvic region was created by blunt dissection, carefully separating the skin from the fascie. A polyurethane sponge with the dimension of 8x5x3 mm containing a slow-release pellet of 400 ng bFGF was placed at the caudal end of the pocket and the incision was closed by a single or double invert suture using Perma-Hand Seide 4/0 (Johnson&Johnson, Brussels, Belgium). The animals were treated by an analgesic subcutaneous injection of carprofen 2 mg/kg.

The angiogenic response was quantitated as previously described (Lichtenberg *et al.,* 1997, 1999 & 2002). Briefly, twenty minutes before euthanasia 1 μCi of $^{125}$I-labelled immunoglobulin (Amersham, UK) in 50 μl of 0.9% NaCl was injected intravenously into a tail vein. The animals were euthanised by $O_2/CO_2$ asphyxiation and the skin overlying the sponge implant was removed. The sponge implant with the pellet was placed in a plastic vial containing 4% formalin

and the $^{125}I$-activity was measured in a γ-counter. Differences in angiogenic response measured as $^{125}I$-activity in cpm were assessed by Student's t-test, grouped data, with P<0.05 regarded as statistically significant. Data was expressed as means ± SEM.

*Mouse metastasis assay*

Female C57BL/6 mice were supplied and delivered by Charles River UK Ltd. The animals were approximately 6 weeks of age at the start of the study. The body weights at the start of dosing were in the range 10-21 g. The mice were housed in solid-bottomed plastic cages, containing wood shavings, in groups of up to 10. During acclimatization, the rooms and cages were cleaned at regular intervals to maintain hygiene. The mice were fed an expanded rodent diet ad libitum and allowed free access to mains tap water. The holding rooms had a 12 h light-dark cycle, and were air-conditioned by a system designed to maintain air temperature within the range 20 ± 3°C (McKay, 2002).

Data was expressed as means ± SEM and analysed using appropriate statistical methods. Statistical significance was assumed when P<0.05.

## BRIEF DESCRIPTION OF THE DRAWINGS

The present invention is further illustrated by reference to the accompanying drawing, in which:

Fig. 1 shows the effects of Compound a on HUVEC (○), fibroblast (▲) and tumor cell (Mel 57(●) and T47D(∇)) proliferation;

Fig. 2 shows the effects of Compound a on 10%, 50% and 100% confluent monolayers of HUVEC. Solid symbols and bars: continuous conditions; open symbols and bars: preincubated conditions.

## EXAMPLES

The invention is further illustrated with reference to the following examples, which are not intended to be in any way limiting to the scope of the invention as claimed. The examples describe test results for the compounds *N*-4-Nitrophenyl-*N'*-[4-bromo-2-(1-*H*-tetrazol-5-yl)phenyl] urea (compound a) and *N*-3,5-Di(trifluoromethyl)phenyl-*N'*-[4-bromo-2-(1-*H*-tetrazol-5-yl)phenyl] urea (compound b).

### Example 1

### Compound a tested in the *in vitro* cell specificity assay

A difference in cell-specificity was observed of Compound a (see Fig. 1). The compound was a more potent inhibitor of the bFGF-induced HUVEC and fibroblast proliferation when compared to the NBCS-induced Mel 57 and T47D proliferation.

### Example 2

### Compound a tested in the *in vitro* cell morphology and proliferation assay

To investigate the mode of action of the compound on proliferating and non-proliferating HUVEC, experiments were performed on 10%, 50% and 100% confluent monolayers of HUVEC. The 10% and 50% confluent HUVEC monolayers represent the status of angiogenic endothelial cells able to proliferate and migrate when stimulated. The 100% HUVEC monolayers represent the quiescent character of the endothelial cells in the existing blood vessel. The 4-hour preincubation experiments were performed to be able to discriminate between general toxicity and the induction of apoptosis.

Compound a was not able to inhibit HUVEC proliferation significantly when added for the 4-hour preincubation period and then removed for the rest of the stimulation period (see Fig. 2). No signs of any cytotoxicity (cell death indicated by the observation of floating cells in the media) was observed during this period. In addition, there was no difference in cell death or any delay of cell growth by the preincubation period during the rest of the 3-day or 5-day incubation period.

However, when the incubation was performed in the continuous presence of the compound, there was a clear inhibitory effect on the proliferation of the cells at the 10% confluence HUVEC monolayers. This effect was observed at the two highest concentrations of the compound, but not at lower concentrations. The compound induced inhibition of HUVEC proliferation was also slightly observed at the 50% confluent monolayers (see Fig. 2). There was no effect of

the compound on the 100% HUVEC monolayers.

This non-cytotoxicity of the compound on the 100% confluent monolayers was confirmed by the fact that there was no change of HUVEC morphology and the amount of floating dead cells in the culture medium observed during the culture periods with the compound.

**Example 3**

**Compounds a and b tested in the *in vivo* mouse anti-angiogenesis assay**

Three separate experiments were conducted: Two experiments were performed with Compound a and one experiment was performed with Compound b. In the first two experiments with Compound a, 3 groups each comprising 5 animals was treated orally with Compound a at the dose levels of 0 (saline), 5 and 10 mg/kg/day (experiment 1) and with 20 and 40 mg/kg/day (experiment 2). In the third experiment, 3 groups each comprising 6 animals was treated orally with the vehicle (saline) and 80 mg/kg/day of Compound a. In all experiments the animals were treated from Days 3-9 and killed at day 10.

Compound a elicited a dose-response relationship at all doses. At 10 and 40 mg/kg/day a significant inhibition of the neovacularisation of 37% and 48% was obtained (see Table 1). A dose of 5 mg/kg/day appeared to be the No Effect Level (NOEL).

Compound b at a dose of 80 mg/kg/day inhibited the angiogenesis response of approximately 60% compared to vehicle treated animals. The selected dose levels were well tolerated by the mice; no signs of toxicity or changes of the body weight gain were observed (data not shown).

**Table 1**

The effect of Compounds a and b on the neovascularisation in mice (expressed as percent inhibition)

| **Dose mg/kg→ Treatment↓** | **5** | **10** | **20** | **40** | **80** |
|---|---|---|---|---|---|
| **Compound a** | 15% | 37%* | 28% | 48%* | |
| **Compound b** | | | | | 61%* |

* $P < 0.05$, compared to vehicle (t-test)

**Example 4**

**Compounds a and b tested in the *in vivo* mouse metastasis assay**

Two separate studies were conducted: One was performed with Compound a (experiment 1) and one with Compound b (experiment 2). In each study there were 4 treatment groups. The treatment groups were as follows:

| **Group** | **Treatment** | **Dose** |
|---|---|---|
| 1 | Untreated Control | - |
| 2 | Vehicle Control | 20 ml/kg p.o. |
| 3 | Compounds a or b | 60 mg/kg p.o. |
| 4 | Compounds a or b | 80 mg/kg p.o. |

Treatments for groups 2-4 were administered orally, by gavage. The dose volume used was 20 ml/kg for groups 2 and 4 and 15 ml/kg for group 3.

C57BU6 mice were injected intravenously, via a tail vein, with 0.1 ml of a suspension of B16/F10 melanoma cells (approximately 3 x $10^5$ cells/mouse) on day 0. With the exception of the untreated group, animals were dosed orally, by gavage, or intravenously, according to their allocated treatment group once daily, from day -2 to day 10 (13 administrations). The animals were killed on day 14 (14 days after injection of the tumour cells). The lungs from each animal were removed and weighed prior to fixing in Bouin's solution. After fixation, the number of colonies on the surface of each set of lungs was counted by eye and these data were used for statistical analysis.

Oral administration of Compound a, at doses of 60 and 80 mg/kg, resulted in a significant reduction of 17% and 21%, respectively in the number of melanoma lung colonies when compared to vehicle treated mice (see Table 2).

Oral administration of Compound b, at similar doses (60 and 80 mg/kg), produced a significant reduction of 36% and 44%, respectively in the number of melanoma lung colonies compared to vehicle treated mice (see Table 3).

**Table 2**

The effect of Compound a on the development of B16 melanoma lung colonies in C57BU6 mice

| Group | Treatment | Colony Count | % Reduction |
|---|---|---|---|
| 1 | Untreated Control | - | - |
| 2 | Vehicle Control (20 ml/kg) | 78.29 ± 3.56 | - |
| 3 | Compound a (60 mg/kg) | 64.68 ± 2.65* | 17.38 |
| 4 | Compound a (80 mg/kg) | 61.85 ± 2.89* | 21.00 |
| * $P$ < 0.01, compared to vehicle (Kruskal-Wallis and Dunnett's test) | | | |

**Table 3**

The effect of Compound b on the development of B16 melanoma lung colonies in C57BL/6 mice

| Group | Treatment | Colony Count | % Reduction |
|---|---|---|---|
| 1 | Untreated Control | - | - |
| 2 | Vehicle Control (20 ml/kg) | 65.40 ± 7.90 | - |
| 3 | Compound b (60 mg/kg) | 41.55± 9.55* | 36.45 |
| 4 | Compound b (80 mg/kg) | 36.65 ± 5.82* | 43.97 |
| * $P$ < 0.01, compared to vehicle (Kruskal-Wallis and Dunnett's test) | | | |

## Claims

**1.** The use of a compound of general formula I

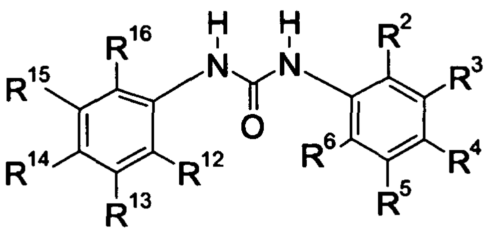

(I)

or a pharmaceutically acceptable salt thereof
wherein $R^2$ represents tetrazolyl; and

- $R^3$, $R^4$, $R^5$, $R^6$, $R^{12}$, $R^{13}$, $R^{14}$, $R^{15}$, and $R^{16}$ independently of each other represent hydrogen, halo, trifluoromethyl, nitro, alkyl, alkylcarbonyl, $-NR^aR^b$, $-NR^a-CO-R^b$, phenyl or heteroaryl;
  which phenyl is optionally substituted with halo, trifluoromethyl, nitro, $-CO-NHR^c$, $-CO-O-R^c$ or -CO-NR'R";
  wherein $R^c$ is hydrogen, alkyl, or phenyl;
  R' and R" independently of each other are hydrogen or alkyl; or
  R' and R" together with the nitrogen to which they are attached form a 5- to 7-membered heterocyclic ring, which ring may optionally comprise as a ring member, one oxygen atom, and/or one additional nitrogen atom, and/or one carbon-carbon double bond, and/or one carbon-nitrogen double bond;
  and which heterocyclic ring may optionally be substituted with alkyl;
  $R^a$ and $R^b$ independently of each other are hydrogen or alkyl; or
- $R^{15}$ and $R^{16}$, or $R^{14}$ and $R^{15}$ together with the phenyl ring to which they are attached form a naphthyl ring or an indanyl ring; and $R^3$, $R^4$, $R^5$, $R^6$, $R^{12}$ and $R^{13}$ and the remaining one of $R^{14}$, $R^{15}$ and $R^{16}$ are as defined above;

for the manufacture of a pharmaceutical composition for the treatment, prevention or alleviation of a disease or a disorder or a condition of a mammal, including a human, which disease, disorder or condition is responsive to

inhibition of angiogenesis.

2. The use according to claim 1, wherein

$R^3$, $R^5$, and $R^6$ represent hydrogen; and
$R^4$ represents halo.

3. The use according to claim 1, wherein

$R^3$, $R^5$, and $R^6$ represent hydrogen; and
$R^4$ represents phenyl substituted with trifluoromethyl, nitro or -CO-NHR$^c$;

wherein R$^c$ is phenyl.

4. The use according to claim 1, wherein the compound is

*N*-4-Nitrophenyl-*N*'-[4-bromo-2-(1-*H*-tetrazol-5-yl)phenyl] urea;
*N*-3,5-Di(trifluoromethyl)phenyl-*N*'-[4-bromo-2-(1-*H*-tetrazol-5-yl)phenyl] urea;
*N*-3-Trifluoromethylphenyl-*N*'-[4-(3-nitrophenyl)-2-(1-*H*-tetrazol-5-yl)phenyl] urea;
*N*-3-Trifluoromethylphenyl-*N*'-[4-(4-anilinocarbonylphenyl)-2-(1-*H*-tetrazol-5-yl)phenyl] urea;
*N*-3-Trifluoromethylphenyl-*N'*-[4-(4-trifluoromethylphenyl)-2-(1-*H*-tetrazol-5-yl)phenyl] urea;
*N*-(3-Trifluoromethyl-phenyl)-*N'*-[2-(1-*H*-tetrazol-5-yl)-phenyl] urea;
*N*-(3-Trifluoromethyl-phenyl)-*N'*-[4-bromo-2-(1-*H*-terazol-5-yl)phenyl] urea;
*N*-(3-Trilfuoromethyl-phenyl)-*N'*-[4-phenyl-2-(1-*H*-tetrazol-5-yl)-phenyl] urea;
*N*-(3-Chloro-phenyl)-*N'*-[2-(1-*H*-tetrazol-5-yl)-phenyl] urea;
*N*-(3-Trifluoromethyl-phenyl)-*N'*-[4-amino-2-(1-*H*-tetrazol-5-yl)-phenyl] urea;
*N*-(3-Trifluoromethyl-phenyl)-*N'*-[4-acetylamino-2-(1-*H*-tetrazol-5-yl)phenyl] urea;
*N*-(3-Trilfuoromethyl-phenyl)-*N'*-[4-carbamoyl-2-(1-*H*-tetrazol-5-yl)-phenyl] urea;
*N*-(3-Trifluoromethyl-phenyl)-*N'*-[4-(*N",N"*-dimethylcarbamoyl)-2-(1-*H*-tetrazol-5-yl)-phenyl] urea;
3'-(1-*H*-tetrazol-5-yl)-4'-[3-(3-trifluoromethyl-phenyl)-ureido]-biphenyl-4-carboxylic acid;
*N*-(Indan-5-yl)-*N'*-[2-(1-*H*-tetrazol-5-yl)-phenyl] urea;
N-(Biphenyl-4-yl)-*N'*-[2-(1-*H*-tetrazol-5-yl)-phenyl] urea;
*N*-(Biphenyl-3-yl)-*N'*-[2-(1-*H*-tetrazol-5-yl)-phenyl] urea;
*N*-(3-Acetyl-phenyl)-*N'*-[2-(1-*H*-tetrazol-5-yl)-phenyl] urea;
*N*-(Biphenyl-3-yl)-*N'*-[2-(1-*H*-tetrazol-5-yl)-phenyl] urea;
*N*-[3-(Pyridin-3-yl)-phenyl]-*N'*-[2-(1-*H*-tetrazol-5-yl)-phenyl] urea;
*N*-(3-Bromo-phenyl)-*N'*-[4'-(4-methyl-piperazine-1-carbonyl)-3-(1-*H*-tetrazol-5-yl)-biphenyl-4-yl] urea;
*N*-(3,5-Dichloro-phenyl)-*N'*-[4-bromo-2-(1-*H*-tetrazol-5-yl)-phenyl] urea;
*N*-(3,4-Dichloro-phenyl)-*N'*-[4-bromo-2-(1-*H*-tetrazol-5-yl)-phenyl] urea;
*N*-(Naphthalen-1-yl)-*N'*-[4-bromo-2-(1-*H*-tetrazol-5-yl)-phenyl] urea;
*N*-(2-Trifluoromethyl-phenyl)-*N'*-[4-bromo-2-(1-*H*-tetrazol-5-yl)-phenyl] urea;
*N*-(2-Fluoro-phenyl)-*N'*-[4-bromo-2-(1-*H*-tetrazol-5-yl)-phenyl] urea;
*N*-(2-Ethyl-phenyl)-*N'*-[4-bromo-2-(1-*H*-tetrazol-5-yl)-phenyl] urea;

or a pharmaceutically acceptable salt thereof.

5. The use according to claim 1, wherein the disease, disorder or condition that is responsive to inhibition of angiogenesis is selected from the group consisting of cancer, prostate cancer, lung cancer, breast cancer, bladder cancer, renal cancer, colon cancer, gastric cancer, pancreatic cancer, ovarian cancer, melanoma, hepatoma, sarcoma, lymphoma, exudative macular degeneration, age-related macular degeneration, retinopathy, diabetic retinopathy, proliferative diabetic retinopathy, diabetic macular edema (DME), ischemic retinopathy, retinopathy of prematurity, neovascular glaucoma, corneal neovascularization, rheumatoid arthritis, and psoriasis.

6. The use according to claim 1, wherein the compound is

*N*-4-Nitrophenyl-*N'*-[4-bromo-2-(1-*H*-tetrazol-5-yl)phenyl] urea;
*N*-3,5-Di(trifluoromethyl)phenyl-*N'*-[4-bromo-2-(1-*H*-tetrazol-5-yl)phenyl] urea;
*N*-3-Trifluoromethylphenyl-*N'*-[4-(3-nitrophenyl)-2-(1-*H*-tetrazol-5-yl)phenyl] urea;

*N*-3-Trifluoromethylphenyl-*N'*-[4-(4-anilinocarbonylphenyl)-2-(1-*H*-tetrazol-5-yl)phenyl] urea;
*N*-3-Trifluoromethylphenyl-*N*'-[4-(4-trifluoromethylphenyl)-2-(1-*H*-tetrazol- 5- yl) phenyl] urea;

or a pharmaceutically acceptable salt thereof, and
the treatment is an anti-metastatic treatment.

**7.** The use of a VRAC blocker or a pharmaceutically acceptable salt thereof for the manufacture of a pharmaceutical composition for the treatment, prevention or alleviation of age-related macular degeneration of a mammal, including a human.

**8.** The use according to 7, wherein the VRAC blocker is a compound of general formula I

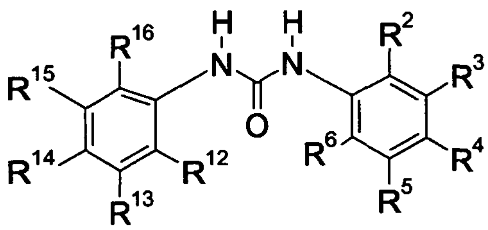

(I)

or a pharmaceutically acceptable salt thereof
wherein $R^2$ represents tetrazolyl; and

- $R^3$, $R^4$, $R^5$, $R^6$, $R^{12}$, $R^{13}$, $R^{14}$, $R^{15}$, and $R^{16}$ independently of each other represent hydrogen, halo, trifluoromethyl, nitro, alkyl, alkylcarbonyl, $-NR^aR^b$, $-NR^a-CO-R^b$, phenyl or heteroaryl;
which phenyl is optionally substituted with halo, trifluoromethyl, nitro, $-CO-NHR^c$, $-CO-O-R^c$ or -CO-NR'R";
wherein $R^c$ is hydrogen, alkyl, or phenyl;
R' and R" independently of each other are hydrogen or alkyl; or
R' and R" together with the nitrogen to which they are attached form a 5- to 7-membered heterocyclic ring, which ring may optionally comprise as a ring member, one oxygen atom, and/or one additional nitrogen atom, and/or one carbon-carbon double bond, and/or one carbon-nitrogen double bond;
and which heterocyclic ring may optionally be substituted with alkyl;
$R^a$ and $R^b$ independently of each other are hydrogen or alkyl; or
- $R^{15}$ and $R^{16}$, or $R^{14}$ and $R^{15}$ together with the phenyl ring to which they are attached form a naphthyl ring or an indanyl ring; and $R^3$, $R^4$, $R^5$, $R^6$, $R^{12}$ and $R^{13}$ and the remaining one of $R^{14}$, $R^{15}$ and $R^{16}$ are as defined above.

**9.** The use according to claim 7, wherein the compound is

*N*-4-Tritrophenyl-*N*'-[4-bromo-2-(1-*H*-tetrazol-5-yl)phenyl] urea;
*N*-3,5-Di(trifluoromethyl)phenyl-*N'*-[4-bromo-2-(1-*H*-tetrazol-5-yl)phenyl] urea;
*N*-3-Trifluoromethylphenyl-N'-[4-(3-nitrophenyl)-2-(1-H-tetrazol-5-yl)phenyl] urea;
*N*-3-Trifluoromethylphenyl-*N*'-[4-(4-anilinocarbonylphenyl)-2-(1-*H*-tetrazol-5-yl)phenyl] urea;
*N*-3-Trifluoromethylphenyl-*N'*-[4-(4-trifluoromethylphenyl)-2-(1-*H*-tetrazol-5-yl)phenyl] urea;
*N*-(3-Trifluoromethyl-phenyl)-*N'*-[2-(1-*H*-tetrazol-5-yl)-phenyl] urea;
*N*-(3-Trifluoromethyl-phenyl)-*N'*-[4-bromo-2-(1-*H*-terazol-5-yl)-phenyl] urea;
*N*-(3-Trilfuoromethyl-phenyl)-*N'*-[4-phenyl-2-(1-*H*-tetrazol-5-yl)phenyl] urea;
*N*-(3-Chloro-phenyl)-*N'*-[2-(1-*H*-tetrazol-5-yl)-phenyl] urea;
*N*-(3-Trifluoromethyl-phenyl)-*N'*-[4-amino-2-(1-*H*-tetrazol-5-yl)-phenyl] urea;
*N*-(3-Trifluoromethyl-phenyl)-*N'*-[4-acetylamino-2-(1-*H*-tetrazol-5-yl)-phenyl] urea;
*N*-(3-Trilfuoromethyl-phenyl)-*N'*-[4-carbamoyl-2-(1-*H*-tetrazol-5-yl)-phenyl] urea;
*N*-(3-Trifluoromethyl-phenyl)-*N'*-[4-(*N",N"*-dimethylcarbamoyl)-2-(1-*H*-tetrazol-5-yl)-phenyl] urea;
3'-(1-*H*-tetrazol-5-yl)-4'-[3-(3-trifluoromethyl-phenyl)-ureido]-biphenyl-4-carboxylic acid;
*N*-(Indan-5-yl)-*N*'-[2-(1-*H*-tetrazol-5-yl)-phenyl] urea;
N-(Biphenyl-4-yl)-*N*'-[2-(1-*H*-tetrazol-5-yl)-phenyl] urea;
*N*-(Biphenyl-3-yl)-*N*'-[2-(1-*H*-tetrazol-5-yl)-phenyl] urea;
*N*-(3-Acetyl-phenyl)-*N*'-[2-(1-*H*-tetrazol-5-yl)-phenyl] urea;

*N*-(Biphenyl-3-yl)-*N'*-[2-(1-*H*-tetrazol-5-yl)-phenyl] urea;
*N*-[3-(Pyridin-3-yl)-phenyl]-*N'*-[2-(1-*H*-tetrazol-5-yl)-phenyl] urea;
*N*-(3-Bromo-phenyl)-*N'*-[4'-(4-methyl-piperazine-1-carbonyl)-3-(1-*H*-tetrazol-5-yl)-biphenyl-4-yl] urea;
*N*-(3,5-Dichloro-phenyl)-*N'*-[4-bromo-2-(1-*H*-tetrazol-5-yl)-phenyl] urea;
*N*-(3,4-Dichloro-phenyl)-*N'*-[4-bromo-2-(1-*H*-tetrazol-5-yl)-phenyl] urea;
*N*-(Naphthalen-1-yl)-*N'*-[4-bromo-2-(1-*H*-tetrazol-5-yl)-phenyl] urea;
*N*-(2-Trifluoromethyl-phenyl)-*N'*-[4-bromo-2-(1-*H*-tetrazol-5-yl)-phenyl] urea;
*N*-(2-Fluoro-phenyl)-*N'*-[4-bromo-2-(1-*H*-tetrazol-5-yl)-phenyl] urea;
*N*-(2-Ethyl-phenyl)-*N'*-[4-bromo-2-(1-*H*-tetrazol-5-yl)-phenyl] urea;

or a pharmaceutically acceptable salt thereof.

## Patentansprüche

**1.** Verwendung einer Verbindung der allgemeinen Formel I

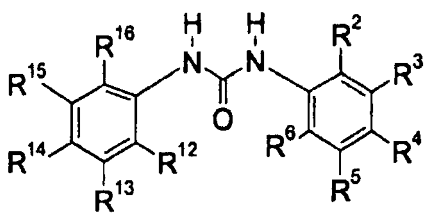

(I)

oder eines pharmazeutisch annehmbaren Salzes davon,
wobei $R^2$ Tetrazolyl darstellt und

- $R^3$, $R^4$, $R^5$, $R^6$, $R^{12}$, $R^{13}$ , $R^{14}$, $R^{15}$ und $R^{16}$ unabhängig voneinander Wasserstoff, Halogen, Trifluormethyl, Nitro, Alkyl, Alkylcarbonyl, $-NR^aR^b$, $-NR^a-CO-R^b$, Phenyl oder Heteroaryl darstellen,
wobei dieses Phenyl wahlweise mit Halogen, Trifluormethyl, Nitro, $-CO-NHR^c$, $-CO-O-R^c$ oder -CO-NR'R" substituiert ist;
wobei $R^c$ Wasserstoff, Alkyl, oder Phenyl bedeutet;
R' und R" unabhängig voneinander Wasserstoff oder Alkyl bedeuten oder
R' und R" zusammen mit dem Stickstoff, an den sie gebunden sind, einen 5-bis 7-gliedrigen heterocyclischen Ring bilden, wobei dieser Ring wahlweise als Ringglied ein Sauerstoffatom und/oder ein zusätzliches Stickstoffatom und/oder eine Kohlenstoff-Kohlenstoff-Doppelbindung und/oder eine Kohlenstoff-Stickstoff-Doppelbindung umfassen kann,
und wobei dieser heterocyclische Ring wahlweise mit Alkyl substituiert sein kann;
$R^a$ und $R^b$ unabhängig voneinander Wasserstoff oder Alkyl bedeuten oder
- $R^{15}$ und $R^{16}$ oder $R^{14}$ und $R^{15}$ zusammen mit dem Phenylring, an den sie gebunden sind, einen Naphthylring oder einen Indanylring bilden und $R^3$, $R^4$, $R^5$, $R^6$, $R^{12}$ und $R^{13}$ und der verbleibende von $R^{14}$, $R^{15}$ und $R^{16}$ wie oben definiert sind,
für die Herstellung einer pharmazeutischen Zusammensetzung für die Behandlung, Verhinderung oder Linderung einer Erkrankung oder einer Störung oder eines Zustands eines Säugers einschließlich eines Menschen, wobei diese Erkrankung, diese Störung oder dieser Zustand auf eine Hemmung der Angiogenese reagiert.

**2.** Verwendung gemäß Anspruch 1, wobei

$R^3$, $R^5$ und $R^6$ Wasserstoff darstellen und
$R^4$ Halogen darstellt.

**3.** Verwendung gemäß Anspruch 1, wobei

$R^3$, $R^5$ und $R^6$ Wasserstoff darstellen und
$R^4$ Phenyl, das mit Trifluormethyl, Nitro oder $-CO-NHR^c$,

wobei $R^c$ Phenyl bedeutet, substituiert ist, darstellt.

**4.** Verwendung gemäß Anspruch 1, wobei die Verbindung

*N*-4-Nitrophenyl-*N*'-[4-brom-2-(1-*H*-tetrazol-5-yl)phenyl]harnstoff;
*N*-3,5-Di(trifluormethyl)phenyl-*N*'-[4-brom-2-(1-*H*-tetrazol-5-yl)phenyl]harnstoff;
*N*-3-Trifluormethylphenyl-*N*'-[4-(3-nitrophenyl)-2-(1-*H*-tetrazol-5-yl)phenyl]harnstoff;
*N*-3-Trifluormethylphenyl-*N*'-[4-(4-anilinocarbonylphenyl)-2-(1-*H*-tetrazol-5-yl)phenyl]harnstoff;
*N*-3-Trifluormethylphenyl-*N*'-[4-(4-trifluormethylphenyl)-2-(1-*H*-tetrazol-5-yl)phenyl]harnstoff;
*N*-(3-Trifluormethyl-phenyl)-*N*'-[2-(1-*H*-tetrazol-5-yl)-phenyl]harnstoff;
*N*-(3-Trifluormethyl-phenyl)-*N*'-[4-brom-2-(1-*H*-tetrazol-5-yl)-phenyl]harnstoff;
*N*-(3-Trifluormethyl-phenyl)-*N*'-[4-phenyl-2-(1-*H*-tetrazol-5-yl)-phenyl]harnstoff;
*N*-(3-Chlor-phenyl)-*N'*-[2-(1-*H*-tetrazol-5-yl)-phenyl]harnstoff;
*N*-(3-Trifluormethyl-phenyl)-*N'*-[4-amino-2-(1-*H*-tetrazol-5-yl)-phenyl]harnstoff;
*N*-(3-Trifluormethyl-phenyl)-*N'*-[4-acetylamino-2-(1-*H*-tetrazol-5-yl)-phenyl]harnstoff;
*N*-(3-Trifluormethyl-phenyl)-*N'*-[4-carbamoyl-2-(1-*H*-tetrazol-5-yl)-phenyl]harnstoff;
*N*-(3-Trifluormethyl-phenyl)-*N'*-[4-(*N''*,*N''*-dimethylcarbamoyl)-2-(1-*H*-tetrazol-5-yl)-phenyl]harnstoff;
3'-(1-*H*-tetrazol-5-yl)-4'-[3-(3-trifluormethyl-phenyl)-ureido]-biphenyl-4-carbonsäure;
*N*-(Indan-5-yl)-*N*'-[2-(1-*H*-tetrazol-5-yl)-phenyl]harnstoff;
*N*-(Biphenyl-4-yl)-*N*'-[2-(1-*H*-tetrazol-5-yl)-phenyl]harnstoff;
*N*-(Biphenyl-3-yl)-*N*'-[2-(1-*H*-tetrazol-5-yl)-phenyl]harnstoff;
*N*-(3-Acetyl-phenyl)-*N*'-[2-(1-*H*-tetrazol-5-yl)-phenyl]harnstoff;
*N*-(Biphenyl-3-yl)-*N*'-[2-(1-*H*-tetrazol-5-yl)-phenyl]harnstoff;
*N*-[3-(Pyridin-3-yl)-phenyl]-*N*'-[2-(1-*H*-tetrazol-5-yl)-phenyl]harnstoff;
*N*-(3-Brom-phenyl)-*N*'-[4'-(4-methyl-piperazin-1-carbonyl)-3-(1-*H*-tetrazol-5-yl)-biphenyl-4-yl]harnstoff;
*N*-(3,5-Dichlor-phenyl)-*N*'-[4-brom-2-(1-*H*-tetrazol-5-yl)-phenyl]harnstoff;
*N*-(3,4-Dichlor-phenyl)-*N'*-[4-brom-2-(1-*H*-tetrazol-5-yl)-phenyl]harnstoff;
*N*-(Naphthalin-1-yl)-*N'*-[4-brom-2-(1-*H*-tetrazol-5-yl)-phenyl]harnstoff;
*N*-(2-Trifluormethyl-phenyl)-*N'*-[4-brom-2-(1-*H*-tetrazol-5-yl)-phenyl]harnstoff;
*N*-(2-Fluor-phenyl)-*N'*-[4-brom-2-(1-*H*-tetrazol-5-yl)-phenyl]harnstoff;
*N*-(2-Ethyl-phenyl)-*N'*-[4-brom-2-(1-*H*-tetrazol-5-yl)-phenyl]harnstoff;

oder ein pharmazeutisch annehmbares Salz davon ist.

**5.** Verwendung gemäß Anspruch 1, wobei die Erkrankung, die Störung oder der Zustand, die oder der auf eine Hemmung der Angiogenese reagiert, aus der Gruppe bestehend aus Krebs, Prostatakrebs, Lungenkrebs, Brustkrebs, Blasenkrebs, Nierenkrebs, Dickdarmkrebs, Magenkrebs, Bauchspeicheldrüsenkrebs, Eierstockkrebs, Melanom, Hepatom, Sarkom, Lymphom, exsudativer Makuladegeneration, altersabhängiger Makuladegeneration, Retinopathie, diabetischer Retinopathie, proliferativer diabetischer Retinopathie, diabetischem Makulaödem (DME), ischämischer Retinopathie, Frühgeborenenretinopathie, neovaskulärem Glaukom, Hornhaut-Neovaskularisation, rheumatoider Arthritis und Psoriasis ausgewählt ist.

**6.** Verwendung gemäß Anspruch 1, wobei die Verbindung

*N*-4-Nitrophenyl-*N*'-[4-brom-2-(1-*H*-tetrazol-5-yl)phenyl]harnstoff;
*N*-3,5-Di(trifluormethyl)phenyl-*N*'-[4-brom-2-(1-*H*-tetrazol-5-yl)phenyl]harnstoff;
*N*-3-Trifluormethylphenyl-*N*'-[4-(3-nitrophenyl)-2-(1-*H*-tetrazol-5-yl)phenyl]harnstoff;
*N*-3-Trifluormethylphenyl-*N*'-[4-(4-anilinocarbonylphenyl)-2-(1-*H*-tetrazol-5-yl)phenyl]harnstoff;
*N*-3-Trifluormethylphenyl-*N'*-[4-(4-trifluormethylphenyl)-2-(1-*H*-tetrazol- 5- yl) phenyl] harnstoff

oder ein pharmazeutisch annehmbares Salz davon ist und
die Behandlung eine antimetastatische Behandlung ist.

**7.** Verwendung eines VRAC-Blockers oder eines pharmazeutisch annehmbaren Salzes davon für die Herstellung einer pharmazeutischen Zusammensetzung für die Behandlung, Verhinderung oder Linderung von altersabhängiger Makuladegeneration bei einem Säuger einschließlich eines Menschen.

**8.** Verwendung gemäß Anspruch 7, wobei der VRCA-Blocker eine Verbindung der allgemeinen Formel I

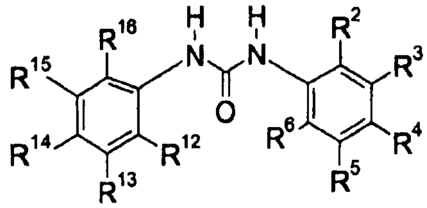

(I)

oder ein pharmazeutisch annehmbares Salz davon ist,
wobei $R^2$ Tetrazolyl darstellt und

- $R^3$, $R^4$, $R^5$, $R^6$, $R^{12}$, $R^{13}$, $R^{14}$, $R^{15}$ und $R^{16}$ unabhängig voneinander Wasserstoff, Halogen, Trifluormethyl, Nitro, Alkyl, Alkylcarbonyl, $-NR^aR^b$, $-NR^a-CO-R^b$, Phenyl oder Heteroaryl darstellen,
wobei dieses Phenyl wahlweise mit Halogen, Trifluormethyl, Nitro, $-CO-NHR^c$, $-CO-O-R^c$ oder -CO-NR'R" substituiert ist;
wobei $R^c$ Wasserstoff, Alkyl, oder Phenyl bedeutet;
R' und R" unabhängig voneinander Wasserstoff oder Alkyl bedeuten oder
R' und R" zusammen mit dem Stickstoff, an den sie gebunden sind, einen 5-bis 7-gliedrigen heterocyclischen Ring bilden, wobei dieser Ring wahlweise als Ringglied ein Sauerstoffatom und/oder ein zusätzliches Stickstoffatom und/oder eine Kohlenstoff-Kohlenstoff-Doppelbindung und/oder eine Kohlenstoff-Stickstoff-Doppelbindung umfassen kann,
und wobei dieser heterocyclische Ring wahlweise mit Alkyl substituiert sein kann;
$R^a$ und $R^b$ unabhängig voneinander Wasserstoff oder Alkyl bedeuten oder
- $R^{15}$ und $R^{16}$ oder $R^{14}$ und $R^{15}$ zusammen mit dem Phenylring, an den sie gebunden sind, einen Naphthylring oder einen Indanylring bilden und $R^3$, $R^4$, $R^5$, $R^6$, $R^{12}$ und $R^{13}$ und der verbleibende von $R^{14}$, $R^{15}$ und $R^{16}$ wie oben definiert sind.

**9.** Verwendung gemäß Anspruch 7, wobei die Verbindung

*N*-4-Nitrophenyl-*N*'-[4-brom-2-(1-*H*-tetrazol-5-yl)phenyl]harnstoff ;
*N*-3,5-Di(trifluormethyl)phenyl-*N*'-[4-brom-2-(1-*H*-tetrazol-5-yl)phenyl]harnstoff ;
*N*-3-Trifluormethylphenyl-*N*'-[4-(3-nitrophenyl)-2-(1-*H*-tetrazol-5-yl)phenyl]harnstoff;
*N*-3-Trifluormethylphenyl-*N*'-[4-(4-anilinocarbonylphenyl)-2-(1-*H*-tetrazol-5-yl)phenyl]harnstoff;
*N*-3-Trifluormethylphenyl-*N*'-[4-(4-trifluormethylphenyl)-2-(1-*H*-tetrazol-5-yl)phenyl]harnstoff;
*N*-(3-Trifluormethyl-phenyl)-*N*'-[2-(1-*H*-tetrazol-5-yl)-phenyl]harnstoff;
*N*-(3-Trifluormethyl-phenyl)-*N*'-[4-brom-2-(1-*H*-tetrazol-5-yl)-phenyl]harnstoff;
*N*-(3-Trifluormethyl-phenyl)-*N*'-[4-phenyl-2-(1-*H*-tetrazol-5-yl)-phenyl]harnstoff;
*N*-(3-Chlor-phenyl)-*N'*-[2-(1-*H*-tetrazol-5-yl)-phenyl]harnstoff;
*N*-(3-Trifluormethyl-phenyl)-*N'*-[4-amino-2-(1-*H*-tetrazol-5-yl)-phenyl]harnstoff;
*N*-(3-Trifluormethyl-phenyl)-*N'*-[4-acetylamino-2-(1-*H*-tetrazol-5-yl)-phenyl]harnstoff;
*N*-(3-Trifluormethyl-phenyl)-*N'*-[4-carbamoyl-2-(1-*H*-tetrazol-5-yl)-phenyl]harnstoff;
*N*-(3-Trifluormethyl-phenyl)-*N'*-[4-(*N"*,*N"*-dimethylcarbamoyl)-2-(1-*H*-tetrazol-5-yl)-phenyl]harnstoff;
3'-(1-*H*-tetrazol-5-yl)-4'-[3-(3-trifluormethyl-phenyl)-ureido]-biphenyl-4-carbonsäure;
*N*-(Indan-5-yl)-*N'*-[2-(1-*H*-tetrazol-5-yl)-phenyl]harnstoff;
*N*-(Biphenyl-4-yl)-*N'*-[2-(1-*H*-tetrazol-5-yl)-phenyl]harnstoff;
*N*-(Biphenyl-3-yl)-*N*'-[2-(1-*H*-tetrazol-5-yl)-phenyl]harnstoff;
*N*-(3-Acetyl-phenyl)-*N*'-[2-(1-*H*-tetrazol-5-yl)-phenyl]harnstoff;
*N*-(Biphenyl-3-yl)-*N*'-[2-(1-*H*-tetrazol-5-yl)-phenyl]harnstoff;
*N*-[3-(Pyridin-3-yl)-phenyl]-*N*'-[2-(1-*H*-tetrazol-5-yl)-phenyl]harnstoff;
*N*-(3-Brom-phenyl)-*N'*-[4'-(4-methyl-piperazin-1-carbonyl)-3-(1-*H*-tetrazol-5-yl)-biphenyl-4-yl]harnstoff;
*N*-(3,5-Dichlor-phenyl)-*N'*-[4-brom-2-(1-*H*-tetrazol-5-yl)-phenyl]harnstoff;
*N*-(3,4-Dichlor-phenyl)-N'-[4-brom-2-(1-*H*-tetrazol-5-yl)-phenyl]harnstoff;
*N*-(Naphthalin-1-yl)-*N'*-[4-brom-2-(1-*H*-tetrazol-5-yl)-phenyl]harnstoff;
*N*-(2-Trifluormethyl-phenyl)-*N'*-[4-brom-2-(1-*H*-tetrazol-5-yl)-phenyl]harnstoff;
*N*-(2-Fluor-phenyl)-*N'*-[4-brom-2-(1-*H*-tetrazol-5-yl)-phenyl]harnstoff;
*N*-(2-Ethyl-phenyl)-*N'*-[4-brom-2-(1-*H*-tetrazol-5-yl)-phenyl]harnstoff;

oder ein pharmazeutisch annehmbares Salz davon ist.

## Revendications

**1.** Utilisation d'un composé de formule générale I

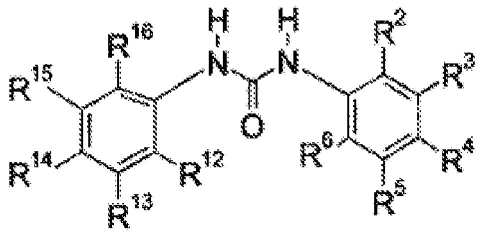

(I)

ou d'un sel pharmaceutiquement acceptable de celui-ci
dans laquelle $R^2$ représente un tétrazolyle ; et

- $R^3$, $R^4$, $R^5$, $R^6$, $R^{12}$, $R^{13}$, $R^{14}$, $R^{15}$, et $R^{16}$ indépendamment les uns des autres représentent un atome hydrogène, halo, trifluorométhyle, nitro, alkyle, alkylcarbonyle, $-NR^aR^b$, $-NR^a-CO-R^b$, phényle ou hétéroaryle ;
lequel phényle est éventuellement substitué par un halo, trifluorométhyle, nitro, $-CO-NHR^c$, $-CO-O-R^c$ ou -CO-NR'R" ;
dans lesquels $R^c$ est un hydrogène, alkyle, ou phényle ;
R' et R" indépendamment l'un de l'autre sont un hydrogène ou un alkyle ; ou
R' et R" conjointement avec l'azote auquel ils sont liés forment un cycle hétérocyclique à 5 à 7 chaînons, lequel cycle peut éventuellement comprendre un élément cyclique, un atome d'oxygène, et/ou un atome d'azote supplémentaire, et/ou une double liaison carbone-carbone, et/ou une double liaison carbone-azote ;
et lequel cycle hétérocyclique peut éventuellement être substitué par un alkyle ;
$R^a$ et $R^b$ indépendamment l'un de l'autre sont un hydrogène ou alkyle ; ou
- $R^{15}$ et $R^{16}$, ou $R^{14}$ et $R^{15}$ conjointement avec le cycle phényle auquel ils sont liés forment un cycle naphtyle ou un cycle indanyle ; et $R^3$, $R^4$, $R^5$, $R^6$, $R^{12}$ et $R^{13}$ et celui restant de $R^{14}$, $R^{15}$ et $R^{16}$ sont tels que définis ci-dessus ;
pour la préparation d'une composition pharmaceutique destinée au traitement, à la prévention ou au soulagement d'une maladie ou d'un trouble ou d'une condition d'un mammifère, y compris d'un être humain, laquelle maladie, lequel trouble ou laquelle condition est sensible à l'inhibition de l'angiogenèse.

**2.** Utilisation selon la revendication 1, dans laquelle :

$R^3$, $R^5$, et $R^6$ représentent un hydrogène ; et
$R^4$ représente un halo.

**3.** Utilisation selon la revendication 1, dans laquelle :

$R^3$, $R^5$, et $R^6$ représentent un hydrogène ; et
$R^4$ représente un phényle substitué par un trifluorométhyle, nitro ou $-CO-NHR^c$ ;

dans lequel $R^c$ est un phényle.

**4.** Utilisation selon la revendication 1, dans laquelle le composé est

la *N*-4-nitrophényl-*N*'-[4-bromo-2-(1-*H*-tétrazol-5-yl)phényl]urée ;
la *N*-3,5-di (trifluorométhyl)phényl-*N*'-[4-bromo-2-(1-*H*-tétrazol-5-yl)phényl] urée ;
la *N*-3-trifluorométhylphényl-*N*'-[4-(3-nitrophényl) -2-(1-*H*-tétrazol-5-yl)phényl] urée ;
la *N*-3-trifluorométhylphényl-*N'*-[4-(4-anilinocarbonylphényl)-2-(1-*H*-tétrazol-5-yl)phényl] urée ;

la *N*-3-trifluorométhylphényl-*N*'-[4-(4-trifluorométhylphényl)-2-(1-*H*-tétrazol-5-yl)phényl] urée ;
la *N*-(3-trifluorométhyl-phényl)-*N*'-[2-(1-*H*-tétrazol-5-yl)-phényl] urée ;
la *N*-(3-trifluorométhyl-phényl)-*N*'-[4-bromo-2-(1-H-tétrazol-5-yl)-phényl] urée ;
la *N*-(3-trifluorométhyl-phényl)-*N*'-[4-phényl-2-(1-H-tétrazol-5-yl)-phényl] urée ;
la *N*- (3-chloro-phényl)-*N*'-[2-(1-*H*-tétrazol-5-yl)-phényl] urée ;
la *N*-(3-trifluorométhyl-phényl)-*N*'-[4-amino-2-(1-H-tétrazol-5-yl)-phényl] urée ;
la *N*-(3-trifluorométhyl-phényl)-*N'*-[4-acétylamino-2-(1-H-tétrazol-5-yl)-phényl] urée ;
la *N*-(3-trifluorométhyl-phényl)-*N*'-[4-carbamoyl-2-(1-*H*-tétrazol-5-yl)-phényl] urée ;
la *N*- (3-trifluorométhyl-phényl) -*N'*- [4- *(N",N"*-diméthylcarbamoyl)-2-(1-*H*-tétrazol-5-yl)-phényl] urée ;
l'acide 3'-(1-*H*-tétrazol-5-yl)-4'-[3-(3-trifluorométhyl-phényl)-uréido]-biphényl-4-carboxylique ;
la *N*-(indan-5-yl)-*N'*-[2-(1-*H*-tétrazol-5-yl)-phényl] urée ;
la *N*-(biphényl-4-yl)-*N'*-[2-(1-*H*-tétrazol-5-yl)-phényl] urée ;
la *N*-(biphényl-3-yl)-*N*'- [2-(1-*H*-tétrazol-5-yl)-phényl] urée ;
la *N*-(3-acétyl-phényl)-*N*'- [2-(1-*H*-tétrazol-5-yl)-phényl] urée ;
la *N*-(biphényl-3-yl)-*N'*-[2-(1-*H*-tétrazol-5-yl)-phényl] urée ;
la *N*-[3-(pyridin-3-yl)-phényl]-*N*'-[2-(1-*H*-tétrazol-5-yl)-phényl] urée ;
la *N*-(3-bromo-phényl)-*N'*-[4'-(4-méthyl-pipérazine-1-carbonyl)-3-(1-*H*-tétrazol-5-yl)-biphényl-4-yl] urée ;
la *N*-(3,5-dichloro-phényl)-*N'*-[4-bromo-2-(1-*H*-tétrazol-5-yl)-phényl] urée ;
la *N*-(3,4-dichloro-phényl)-*N'*-[4-bromo-2-(1-*H*-tétrazol-5-yl)-phényl] urée ;
la *N*-(naphthalèn-1-yl)-*N'*-[4-bromo-2-(1-*H*-tétrazol-5-yl)-phényl] urée ;
la *N*-(2-trifluorométhyl-phényl)-*N'*-[4-bromo-2-(1-H-tétrazol-5-yl)-phényl] urée ;
la *N*-(2-fluoro-phényl)-*N'*-[4-bromo-2-(1-*H*-tétrazol-5-yl)-phényl] urée ;
la *N*-(2-éthyl-phényl)-*N'*-[4-bromo-2-(1-*H*-tétrazol- 5- yl)-phényl] urée ;

ou un sel pharmaceutiquement acceptable de ceux-ci.

**5.** Utilisation selon la revendication 1, dans laquelle la maladie, le trouble ou la condition qui est sensible à l'inhibition de l'angiogenèse est choisi(e) dans le groupe constitué par un cancer, le cancer de la prostate, le cancer du poumon, le cancer du sein, le cancer de la vessie, le cancer du rein, le cancer du côlon, un cancer gastrique, le cancer du pancréas, le cancer des ovaires, un mélanome, un hépatome, un sarcome, un lymphome, la dégénérescence maculaire exsudative, la dégénérescence maculaire liée à l'âge, une rétinopathie, une rétinopathie diabétique, une rétinopathie diabétique proliférative, un oedème maculaire diabétique (DME), une rétinopathie ischémique, une rétinopathie de prématurité, un glaucome néovasculaire, la néovascularisation de la cornée, la polyarthrite rhumatoïde, et le psoriasis.

**6.** Utilisation selon la revendication 1, dans laquelle le composé est

la *N*-4-nitrophényl-*N'*-[4-bromo-2-(1-*H*-tétrazol-5-yl)phényl]urée ;
la *N*-3,5-di(trifluorométhyl)phényl-*N'*-[4-bromo-2-(1-*H*-tétrazol-5-yl)phényl] urée ;
la *N*-3-trifluorométhylphényl-*N'*-[4-(3-nitrophényl) -2-(1-*H*-tétrazol-5-yl)phényl] urée ;
la *N*-3-trifluorométhylphényl-*N'*-[4-(4-anilinocarbonylphényl)-2-(1-*H*-tétrazol-5-yl)phényl] urée ;
la *N*-3-trifluorométhylphényl-*N'*-[4-(4-trifluorométhylphényl)-2-(1-*H*-tétrazol- 5- yl) phényl] urée ;

ou un sel pharmaceutiquement acceptable de ceux-ci, et
le traitement est un traitement anti-métastasique.

**7.** Utilisation d'un bloqueur des canaux VRAC ou d'un sel pharmaceutiquement acceptable de celui-ci pour la préparation d'une composition pharmaceutique destinée au traitement, à la prévention ou au soulagement de la dégénérescence maculaire liée à l'âge chez un mammifère, y compris chez un être humain.

**8.** Utilisation selon la revendication 7, dans laquelle le bloqueur des canaux VRAC est un composé de formule générale I

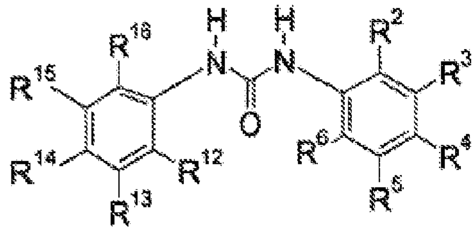

(I)

ou un sel pharmaceutiquement acceptable de celui-ci
dans laquelle $R^2$ représente un tétrazolyle ; et

- $R^3$, $R^4$, $R^5$, $R^6$, $R^{12}$, $R^{13}$, $R^{14}$, $R^{15}$, et $R^{16}$ indépendamment les uns des autres représentent un hydrogène, halo, trifluorométhyle, nitro, alkyle, alkylcarbonyle, $-NR^aR^b$, $-NR^a-CO-R^b$, phényle ou hétéroaryle ; lequel phényle est éventuellement substitué par un halo, trifluorométhyle, nitro, $-CO-NHR^c$, $-CO-O-R^c$

ou -CO-NR'R" ;
dans lesquels $R^c$ est un hydrogène, alkyle, ou phényle ;
R' et R" indépendamment l'un de l'autre sont un hydrogène ou alkyle ; ou
R' et R" conjointement avec l'azote auquel ils sont liés forment un cycle hétérocyclique à 5 à 7 chaînons, lequel cycle peut éventuellement comprendre un élément cyclique, un atome d'oxygène, et/ou un atome d'azote supplémentaire, et/ou une double liaison carbone-carbone, et/ou une double liaison carbone-azote ;
et lequel cycle hétérocyclique peut éventuellement être substitué par un alkyle ;
$R^a$ et $R^b$ indépendamment l'un de l'autre sont un hydrogène ou alkyle ; ou

- $R^{15}$ et $R^{16}$, ou $R^{14}$ et $R^{15}$ conjointement avec le cycle phényle auquel ils sont liés forment un cycle naphtyle ou un cycle indanyle ; et $R^3$, $R^4$, $R^5$, $R^6$, $R^{12}$ et $R^{13}$ et celui restant de $R^{14}$, $R^{15}$ et $R^{16}$ sont tels que définis ci-dessus.

**9.** Utilisation selon la revendication 7, dans laquelle le composé est

la *N*-4-nitrophényl-*N'*-[4-bromo-2-(1-*H*-tétrazol-5-yl)phényl]urée ;
la *N*-3,5-di(trifluorométhyl)phényl-*N'*-[4-bromo-2-(1-*H*-tétrazol-5-yl)phényl] urée ;
la *N*-3-trifluorométhylphényl-*N'*-[4-(3-nitrophényl)-2-(1-*H*-tétrazol-5-yl)phényl] urée ;
la *N*-3-trifluorométhylphényl-*N'*-[4-(4-anilinocarbonylphényl)-2-(1-*H*-tétrazol-5-yl)phényl] urée ;
la *N*-3-trifluorométhylphényl-*N*'-[4-(4-trifluorométhylphényl)-2-(1-*H*-tétrazol-5-yl)phényl] urée ;
la *N*-(3-trifluorométhyl-phényl)-*N'*-[2-(1-*H*-tétrazol-5-yl)-phényl] urée ;
la *N*-(3-trifluorométhyl-phényl)-*N'*-[4-bromo-2-(1-H-tétrazol-5-yl)-phényl] urée ;
la *N*-(3-trifluorométhyl-phényl)-*N'*-[4-phényl-2-(1-H-tétrazol-5-yl)-phényl] urée ;
la *N*-(3-chloro-phényl)-*N'*-[2-(1-*H*-tétrazol-5-yl)-phényl] urée ;
la *N*-(3-trifluorométhyl-phényl)-*N'*-[4-amino-2-(1-H-tétrazol-5-yl)-phényl] urée ;
la *N*-(3-trifluorométhyl-phényl)-*N*'-[4-acétylamino-2-(1-H-tétrazol-5-yl)-phényl] urée ;
la *N*-(3-trifluorométhyl-phényl)-*N*'-[4-carbamoyl-2-(1-*H*-tétrazol-5-yl)-phényl] urée ;
la *N*- (3-trifluorométhyl-phényl) -*N'*- [4- *(N", N"*-diméthylcarbamoyl)-2-(1-*H*-tétrazol-5-yl)-phényl] urée ;
l'acide 3'-(1-*H*-tétrazol-5-yl)-4'-[3-(3-trifluorométhyl-phényl)-uréido]-biphényl-4-carboxylique ;
la *N*-(indan-5-yl)-*N'*-[2-(1-*H*-tétrazol-5-yl)-phényl] urée ;
la *N*-(biphényl-4-yl)-*N'*-[2-(1-*H*-tétrazol-5-yl)-phényl] urée ;
la *N*-(biphényl-3-yl)-*N'*-[2-(1-*H*-tétrazol-5-yl)-phényl] urée ;
la *N*-(3-acétyl-phényl)-*N'*-[2-(1-*H*-tétrazol-5-yl)-phényl] urée ;
la *N*-(biphényl-3-yl)-*N'*-[2-(1-*H*-tétrazol-5-yl)-phényl] urée ;
la *N*-[3-(pyridin-3-yl)-phényl]-*N'*-[2-(1-H-tétrazol-5-yl)-phényl] urée ;
la *N*-(3-bromo-phényl)-*N'*-[4'-(4-méthyl-pipérazine-1-carbonyl)-3-(1-*H*-tétrazol-5-yl)-biphényl-4-yl] urée ;
la *N*-(3, 5-dichloro-phényl)-*N'*-[4-bromo-2-(1-*H*-tétrazol-5-yl)-phényl] urée ;
la *N*-(3, 4-dichloro-phényl)-*N'*-[4-bromo-2-(1-*H*-tétrazol-5-yl)-phényl] urée ;
la *N*-(naphthalèn-1-yl)-*N'*-[4-bromo-2-(1-*H*-tétrazol-5-yl)-phényl] urée ;
la *N*-(2-trifluorométhyl-phényl)-*N'*-[4-bromo-2-(1-H-tétrazol-5-yl)-phényl] urée ;
la *N*-(2-fluoro-phényl)-*N'*-[4-bromo-2-(1-*H*-tétrazol-5-yl)-phényl] urée ;

la *N*-(2-éthyl-phényl)-*N'*-[4-bromo-2-(1-*H*-tétrazol- 5- yl)-phényl] urée ;

ou un sel pharmaceutiquement acceptable de ceux-ci.

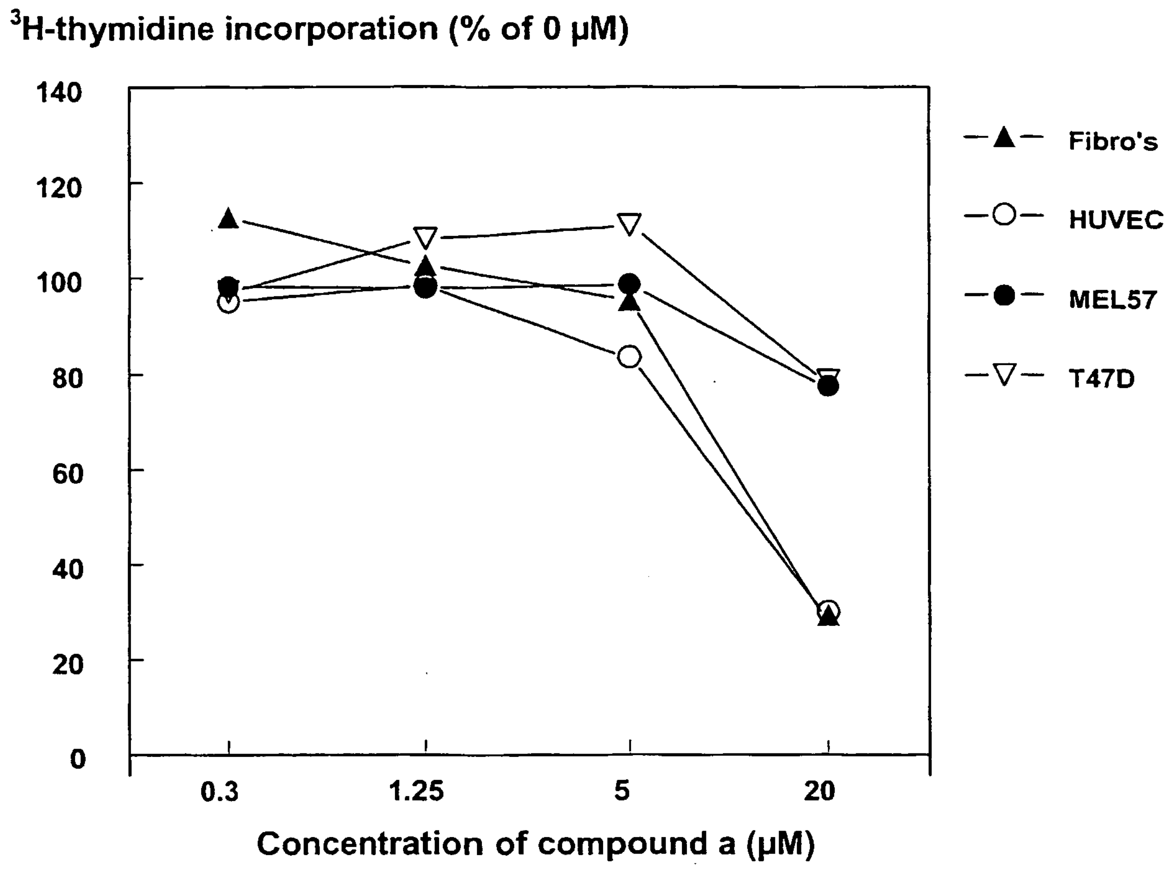
$^3$H-thymidine incorporation (% of 0 μM)
140
120
100
80
60
40
20
0
0.3
1.25
5
20
Concentration of compound a (μM)
Fibro's
HUVEC
MEL57
T47D

Fig. 1

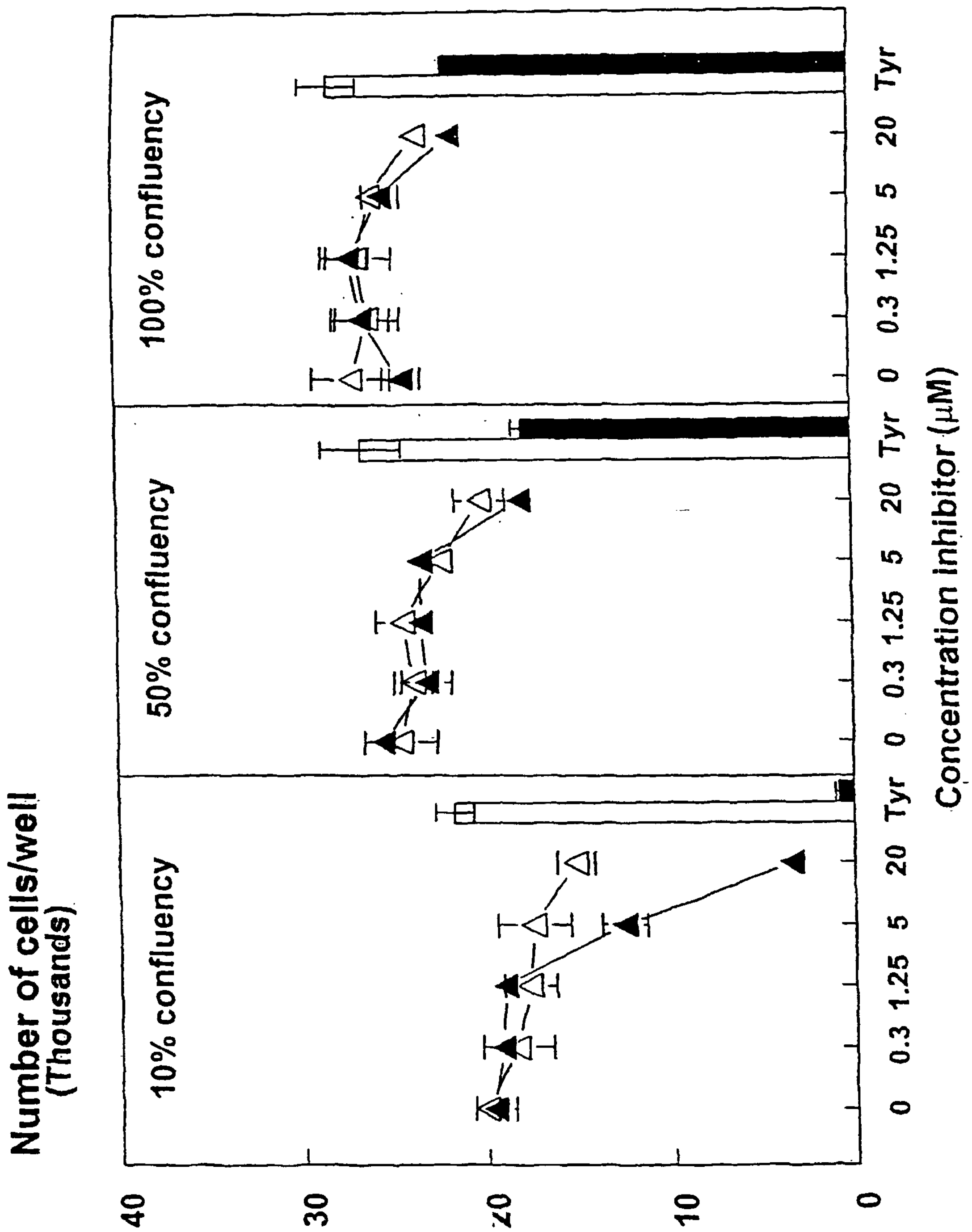
Number of cells/well
(Thousands)
10% confluency
50% confluency
100% confluency
0
0.3
1.25
5
20
Tyr
Concentration Inhibitor (μM)
0
10
20
30
40

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9847879 A
- WO 0024707 A
- WO 0076495 A

### Non-patent literature cited in the description

- Remington's Pharmaceutical Sciences. Maack Publishing Co